(19) Europäisches Patentamt
European Patent Office
Office européen des brevets

(11) EP 2 744 826 B1

(12) # EUROPEAN PATENT SPECIFICATION

(45) Date of publication and mention of the grant of the patent:
**09.02.2022 Bulletin 2022/06**

(21) Application number: **12745883.4**

(22) Date of filing: **14.08.2012**

(51) International Patent Classification (IPC):
***C07K 16/28*** *(2006.01)* ***A61K 39/395*** *(2006.01)*

(52) Cooperative Patent Classification (CPC):
**A61P 35/00; A61P 35/02; C07K 16/2803;**
A61K 2039/505

(86) International application number:
**PCT/EP2012/065904**

(87) International publication number:
**WO 2013/024095 (21.02.2013 Gazette 2013/08)**

(54) **COMBINATION THERAPY WITH AN ANTI-CD19 ANTIBODY AND A PURINE ANALOG**
KOMBINATIONSTHERAPIE MIT EINEM ANTI-CD19 ANTIKÖRPER UND EINEM PURINANALOG
THÉRAPIE COMBINÉE D'UN ANTICORPS ANTI-CD19 AVEC UN ANALOGUE DE PURINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **16.08.2011 EP 11177660**
**16.08.2011 US 201161523862 P**

(43) Date of publication of application:
**25.06.2014 Bulletin 2014/26**

(73) Proprietor: **MorphoSys AG**
**82152 Planegg (DE)**

(72) Inventors:
- **AMERSDORFFER, Jutta**
**85421 Hebertshausen (DE)**
- **STEIDL, Stefan**
**81241 München (DE)**
- **WINDERLICH, Mark**
**81371 München (DE)**
- **KROHN, Susanne**
**81241 München (DE)**
- **ROJKJAER, Lisa**
**CH-8908 Hedingen (CH)**

(74) Representative: **Carpmaels & Ransford LLP**
**One Southampton Row**
**London WC1B 5HA (GB)**

(56) References cited:
**WO-A1-00/74718 WO-A1-2007/064911**
**WO-A1-2007/076950 WO-A1-2011/018225**
**WO-A2-2005/012493 US-A1- 2011 104 150**

- **KOCHENDERFER JAMES N ET AL: "Eradication of B-lineage cells and regression of lymphoma in a patient treated with autologous T cells genetically engineered to recognize CD19", BLOOD, vol. 116, no. 20, November 2010 (2010-11), pages 4099-4102, XP002667047, cited in the application**
- **Rosenberg SA: "Treatment of B Cell Malignancies with T Cells Expressing an Anti-CD19 ChimericReceptor: Assessment of the Impact of Lymphocyte Depletion Prior to T CellTransfer", , September 2008 (2008-09), XP002667046, Retrieved from the Internet: URL:http://www.gemcris.od.nih.gov/Abstracts/940_s.pdf [retrieved on 2012-01-13] cited in the application**
- **SADELAIN MICHEL ET AL: "The promise and potential pitfalls of chimeric antigen receptors", CURRENT OPINION IN IMMUNOLOGY, vol. 21, no. 2, April 2009 (2009-04), pages 215-223, XP002667114, ISSN: 0952-7915 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 744 826 B1

- ESHHAR Z ET AL: "Specific activation and targeting of cytotoxic lymphocytes through chimeric single chains consisting of antibody-binding domains and the gamma or zeta subunits of the immunoglobulin and T-cell receptors", PROCEEDINGS OF THE NATIONAL ACADEMY OF SCIENCES OF USA, NATIONAL ACADEMY OF SCIENCE, WASHINGTON, DC; US, vol. 90, no. 2, 15 January 1993 (1993-01-15), pages 720-724, XP002579936, ISSN: 0027-8424, DOI: 10.1073/PNAS.90.2.720 cited in the application
- SCHEUERMANN R H ET AL: "CD19 antigen in leukemia and lymphoma diagnosis and immunotherapy.", LEUKEMIA & LYMPHOMA AUG 1995 LNKD- PUBMED:8528044, vol. 18, no. 5-6, August 1995 (1995-08), pages 385-397, XP009155469, ISSN: 1042-8194 cited in the application
- MONTSERRAT E ET AL: "Chronic lymphocytic leukaemia treatment", BLOOD REVIEWS, CHURCHILL LIVINGSTONE, vol. 7, no. 3, 1 September 1993 (1993-09-01), pages 164-175, XP022954383, ISSN: 0268-960X, DOI: 10.1016/0268-960X(93)90003-M [retrieved on 1993-09-01] cited in the application

## Description

### Field of the Invention

The present disclosure is related to a pharmaceutical combination of an anti-CD19 antibody and a purine analog for the treatment of non-Hodgkin's lymphoma, chronic lymphocytic leukemia and/or acute lymphoblastic leukemia.

### Background

B cells are lymphocytes that play a large role in the humoral immune response. They are produced in the bone marrow of most mammals, and represent 5-15% of the circulating lymphoid pool. The principal function of B cells is to make antibodies against various antigens, and are an essential component of the adaptive immune system.

Because of their critical role in regulating the immune system, disregulation of B cells is associated with a variety of disorders, such as lymphomas, and leukemias. These include non-Hodgkin's lymphoma (NHL), chronic lymphoid leukemia (CLL) and acute lymphoblastic leukemia (ALL).

NHL is a heterogeneous malignancy originating from lymphocytes. In the United States (U.S.), the incidence is estimated at 65,000/year with mortality of approximately 20,000 (American Cancer Society, 2006; and SEER Cancer Statistics Review). The disease can occur in all ages, the usual onset begins in adults over 40 years, with the incidence increasing with age. NHL is characterized by a clonal proliferation of lymphocytes that accumulate in the lymph nodes, blood, bone marrow and spleen, although any major organ may be involved. The current classification system used by pathologists and clinicians is the World Health Organization (WHO) Classification of Tumours, which organizes NHL into precursor and mature B-cell or T-cell neoplasms. The PDQ is currently dividing NHL as indolent or aggressive for entry into clinical trials. The indolent NHL group is comprised primarily of follicular subtypes, small lymphocytic lymphoma, MALT (mucosa-associated lymphoid tissue), and marginal zone; indolent encompasses approximately 50% of newly diagnosed B-cell NHL patients. Aggressive NHL includes patients with histologic diagnoses of primarily diffuse large B cell (DLBL, DLBCL, or DLCL) (40% of all newly diagnosed patients have diffuse large cell), Burkitt's, and mantle cell. The clinical course of NHL is highly variable. A major determinant of clinical course is the histologic subtype. Most indolent types of NHL are considered to be incurable disease. Patients respond initially to either chemotherapy or antibody therapy and most will relapse. Studies to date have not demonstrated an improvement in survival with early intervention. In asymptomatic patients, it is acceptable to "watch and wait" until the patient becomes symptomatic or the disease pace appears to be accelerating. Over time, the disease may transform to a more aggressive histology. The median survival is 8 to 10 years, and indolent patients often receive 3 or more treatments during the treatment phase of their disease. Initial treatment of the symptomatic indolent NHL patient historically has been combination chemotherapy. The most commonly used agents include: cyclophosphamide, vincristine and prednisone (CVP); or cyclophosphamide, adriamycin, vincristine, prednisone (CHOP). Approximately 70% to 80% of patients will respond to their initial chemotherapy, duration of remissions last on the order of 2-3 years. Ultimately the majority of patients relapse. The discovery and clinical use of the anti-CD20 antibody, rituximab, has provided significant improvements in response and survival rate. The current standard of care for most patients is rituximab + CHOP (R-CHOP) or rituximab + CVP (R-CVP). Interferon is approved for initial treatment of NHL in combination with alkylating agents, but has limited use in the U.S. Rituximab therapy has been shown to be efficacious in several types of NHL, and is currently approved as a first line treatment for both indolent (follicular lymphoma) and aggressive NHL (diffuse large B cell lymphoma). WO2011/018225 discloses an antibody therapy for the treatment of B-cell malignancies comprising a combination of an anti-CD20 antibody with fludarabine. However, there are significant limitations of anti-CD20 monoclonal antibody (mAb), including primary resistance (50% response in relapsed indolent patients), acquired resistance (50% response rate upon re-treatment), rare complete response (2% complete resonse rate in relapsed population), and a continued pattern of relapse. Finally, many B cells do not express CD20, and thus many B-cell disorders are not treatable using anti-CD20 antibody therapy.

In addition to NHL there are several types of leukemias that result from disregulation of B cells. Chronic lymphocytic leukemia (also known as "chronic lymphoid leukemia" or "CLL"), is a type of adult leukemia caused by an abnormal accumulation of B lymphocytes. In CLL, the malignant lymphocytes may look normal and mature, but they are not able to cope effectively with infection. CLL is the most common form of leukemia in adults. Men are twice as likely to develop CLL as women. However, the key risk factor is age. Over 75% of new cases are diagnosed in patients over age 50. More than 10,000 cases are diagnosed every year and the mortality is almost 5,000 a year (American Cancer Society, 2006; and SEER Cancer Statistics Review). CLL is an incurable disease but progresses slowly in most cases. Many people with CLL lead normal and active lives for many years. Because of its slow onset, early-stage CLL is generally not treated since it is believed that early CLL intervention does not improve survival time or quality of life. Instead, the condition is monitored over time. Initial CLL treatments vary depending on the exact diagnosis and the progression of the disease. There are dozens of agents used for CLL therapy. Combination chemotherapy regimens such as FCR (fludarabine, cyclophosphamide and rituximab), and BR (bendamustine and rituximab) are effective in

both newly-diagnosed and relapsed CLL. Allogeneic bone marrow (stem cell) transplantation is rarely used as a first-line treatment for CLL due to its risk.

Another type of leukemia is acute lymphoblastic leukemia (ALL), also known as acute lymphocytic leukemia. ALL is characterised by the overproduction and continuous multiplication of malignant and immature white blood cells (also known as lymphoblasts) in the bone marrow. 'Acute' refers to the undifferentiated, immature state of the circulating lymphocytes ("blasts"), and that the disease progresses rapidly with life expectancy of weeks to months if left untreated. ALL is most common in childhood with a peak incidence of 4-5 years of age. Children of age 12- 16 die more easily from it than others. Currently, at least 80% of childhood ALL are considered curable. Under 4,000 cases are diagnosed every year and the mortality is almost 1,500 a year (American Cancer Society, 2006; and SEER Cancer Statistics Review).

The human CD 19 molecule is a structurally distinct cell surface receptor expressed on the surface of human B cells, including, but not limited to, pre-B cells, B cells in early development {i.e., immature B cells), mature B cells through terminal differentiation into plasma cells, and malignant B cells. CD 19 is expressed by most pre-B acute lymphoblastic leukemias (ALL), non-Hodgkin's lymphomas, B cell chronic lymphocytic leukemias (CLL), pro-lymphocytic leukemias, hairy cell leukemias, common acute lymphocytic leukemias, and some Null-acute lymphoblastic leukemias (Nadler et al, J. Immunol., 131 :244-250 (1983), Loken et al, Blood, 70:1316-1324 (1987), Uckun et al, Blood, 71 :13-29 (1988), Anderson et al, 1984. Blood, 63:1424-1433 (1984), Scheuermann, Leuk. Lymphoma, 18:385-397(1995)). The expression of CD 19 on plasma cells further suggests it may be expressed on differentiated B cell tumors such as multiple myeloma, plasmacytomas, Waldenstrom's tumors (Grossbard et al., Br. J. Haematol, 102:509- 15(1998); Treon et al, Semin. Oncol, 30:248-52(2003)).

Therefore, the CD 19 antigen is a target for immunotherapy in the treatment of non-Hodgkin's lymphoma (including each the subtypes described herein), chronic lymphocytic leukemia and/or acute lymphoblastic leukemia. CD19 has also been suggested as a target for immunotherapy in the treatment of autoimmune disorders in WO2000074718.

Certain CD19 therapies have been shown. T cells expressing an anti-CD19 chimeric antigen receptor (CAR) including both the CD3-4 and CD28 molecules were administered to a patient having follicular lymphoma. Kochenderfer et al., Eradication of B lineage cell and regression of lymphoma in a patient treated with autologous T cells genetically engineered to recognize CD19, Blood, vol. 116, no: 20 (November 2010) utilized anti-CD19 CARTs based on the FMC63 monoclonal antibody. Sadelain et al., The promise and potential pitfalls of chimeric antigen receptors, Current Opinion in Immunology, Elsevier, vol. 21, no.2, 2 April 2009also describes anti-CD19 chimeric antigen receptors (CARs). Rosenberg et al, Treatment of B cell Malignancies with T cells expressing an anti-CD19 chimeric receptor: Assessment of the Impact of Lymphocyte Depletion Prior to T cell Transfer, (September 2008), www.gemcris.od.nih.gov/ Abstracts/940_s.pdf (retrieved on 13 Jan 2012)describes anti-CD19 chimeric antigen receptors (CARs) used with fludarabine. See also Eshhar et al., Proceedings of the National Academy of Sciences of USA, National Academy of Science, Washington, D.C:, vol. 90, no.2 (15 January 1993). Neither Kochenderfer et al., Sadelain et al., Rosenberg et al., nor Eshhar et al., however, describe the antibody specific for CD19 in combination with fludrabine as exemplified herein.

Fludarabine as a therapy in the treatment of CLL was described in Montserrat et al., Chronic lymphocytic leukemia treatment, Blood Review, Churchill Livingstone, vol. 7, no. 3 (1 Sept. 1993), but does not suggest the antibody specific for CD19 in combination with fludrabine as exemplified herein.

The use of a CD19 antibody in B cell disorders is discussed in US 201104150 along with the cursory mention of fludarabine within a long list of potential combination partners, but fails either to teach the exemplified antibody or to suggest the synergistic effects of the combination in the treatment of non-Hodgkin's lymphoma, chronic lymphocytic leukemia and/or acute lymphoblastic leukemia as exemplified herein.

The use of a CD19 antibody in non-specific B cell lymphomas is discussed in WO2007076950 along with the cursory mention of fludarabine within a long list of potential combination partners, but fails either to teach the exemplified antibody or suggest the synergistic effects of the combination in the treatment of non-Hodgkin's lymphoma, chronic lymphocytic leukemia and/or acute lymphoblastic leukemia as exemplified herein.

The use of a CD19 antibody in leukemias and lymphomas is discussed in WO2005012493, along with the cursory mention of fludarabine within a long list of potential combination partners, but fails either to teach the exemplified antibody or suggest the synergistic effects of the combination in the treatment of non-Hodgkin's lymphoma, chronic lymphocytic leukemia and/or acute lymphoblastic leukemia as exemplified herein.

The use of a CD19 antibody in CLL, NHL and ALL is described in Scheuermann et al., CD19 Antigen in Leukemia and Lymphoma Diagnosis and Immunotherapy, Leukemia and Lymphoma, Vol. 18, 385-397 (1995)but fails to suggest the combination exemplified herein.

Additional antibodies specific for CD19 are described in WO2005012493 (US7109304), WO2010053716 (US12/266,999) (Immunomedics); WO2007002223 (US US8097703) (Medarex); WO2008022152 (12/377,251) and WO2008150494 (Xencor), WO2008031056 (US11/852,106) (Medimmune); WO 2007076950 (US 11/648,505 ) (Merck Patent GmbH); WO 2009/052431 (US12/253,895) (Seattle Genetics); and WO2010095031 (12/710,442) (Glenmark Pharmaceuticals).

Combinations of antibodies specific for CD19 and other agents are described in WO2010151341 (US 13/377,514) (The Feinstein Institute); US5686072 (University of Texas), and WO2002022212 (PCT/US01/29026) (IDEC Pharmaceuticals).

It is clear that in spite of the recent progress in the discovery and development of anti-cancer agents, many forms of cancer involving CD19-expressing tumors still have a poor prognosis. Thus, there is a need for improved compositions and methods for treating such forms of cancer.

**Summary**

Neither alone nor in combination does the prior art suggest the synergistic effects of the combination of the exemplified antibody and fludarabine in the treatment of non-Hodgkin's lymphoma, chronic lymphocytic leukemia and/or acute lymphoblastic leukemia.

In one aspect, the present disclosure relates to a synergistic combination of an antibody specific for CD19 and fludarabine for use in the treatment of non-Hodgkin's lymphoma, chronic lymphocytic leukemia or acute lymphoblastic leukemia, wherein the antibody comprises a variable heavy chain of the sequence

EVQLVESGGGLVKPGGSLKLSCAASGYTFTSYVMHWVRQAPGKGLEWIGYINPYNDGTKYNEKFQGRVTISSDK SISTAYMELSSLRSEDTAMYYCARGTYYYGTRVFDYWG QGTLVTVSS (SEQ ID NO: 10) and a variable light chain of the sequence

DIVMTQSPATLSLSPGERATLSCRSSKSLQNVNGNTYLYWFQQKPGQSPQLLIYRMSNLNSGVPDRFSGSGSGT EFTLTISSLEPEDFAVYYCMQHLEYPITFGAGTKLEIK (SEQ ID NO: 11) and a heavy chain constant domain of the sequence

ASTKGPSVFPLAPSSKSTSGGTAALGCLVKDYFPEPVTVSWNSGALTSGVHTFPAVLQSSGLYSLSSVVTVPSSS LGTQTYICNVNHKPSNTKVDKKVEPKSCDKTHTCPPCPAPELLGGPDVFLFPPKPKDTLMISRTPEVTCVVVDVS HEDPEVQFNWYVDGVEVHNAKTKPREEQFNSTFRVVSVLTVVHQDWLNGKEYKCKVSNKALPAPEEKTISKTK GQPREPQVYTLPPSREEMTKNQVSLTCLVKGFYPSDIAVEWESNGQPENNYKTTPPMLDSDGSFFL YSKL TVDKSRWQQGNVFSCSVMH EALHNHYTQKSLSLSPGK(SEQ ID NO: 12).

In one aspect, the present disclosure relates to a synergistic combination of an antibody specific for CD19 and a purine analog. Such combinations are useful in the treatment of B cell malignancies, such as, non-Hodgkin's lymphoma, chronic lymphocytic leukemia and/or acute lymphoblastic leukemia.

In vitro and in vivo models are considered indicative of how a certain compound or combination of compounds would behave in humans. In addition, when compounds are combined either in vitro or in vivo, one expects that the combination has only additive effects. Surprisingly, the inventors found that the combination of a particular antibody specific for CD19 and fludarabine mediated a synergistic level of specific cell killing in a chronic B-cell leukemia cell line (MEC-1) in comparison to the antibody and fludarabine alone. This *in vitro* model is indicative of how the combination will work in the treatment of chronic lymphoid leukemia (CLL) in humans. In addition, and also unexpectedly, the inventors found that the combination of a particular antibody specific for CD19 and fludarabine synergistically inhibited tumor growth and synergistically increased median survival days, both in Burkitt's lymphoma SCID mouse models, in comparison to the antibody and fludarabine alone. These *in vivo* models are indicative of how the combination will work in the treatment of non-Hodgkin's lymphoma in humans. In summary, the combination of the exemplified anti-CD19 antibody and fludarabine behaved synergistically in models relevant to NHL and CLL. As both NHL and CLL are B cell related disorders and CD19 is highly expressed on B-cells, the exemplified combination would have the same mechanism of action and should also behave synergistically in the treatment of other B cell related disorders, e.g. ALL.

Therefore, the combination of the exemplified antibody specific for CD19 and fludarabine will be effective in the treatment of humans in non-Hodgkin's lymphoma, chronic lymphocytic leukemia and/or acute lymphoblastic leukemia. In addition, the antibody specific to CD19 exemplified in the present specification has already entered into clinical trials, where such combinations can be confirmed in humans.

As the mechanism of action of fludarabine and other purine analogs are similar, as purine analogs interfere with the synthesis of nucleic acids, it is believed that synergy should also be seen when treating humans having non-Hodgkin's lymphoma, chronic lymphocytic leukemia and/or acute lymphoblastic leukemia with a combination of the exemplified anti-CD19 antibody and a purine analog other than fludarabine.

As the exemplified anti-CD19 antibody and other anti-CD19 antibodies bind CD19, it is believed that synergy should also be seen when treating humans having non-Hodgkin's lymphoma, chronic lymphocytic leukemia and/or acute lymphoblastic leukemia with a combination of any anti-CD19 antibody and a purine analog, e.g., fludarabine.

As the exemplified anti-CD19 antibody binds a specific epitope of CD19, it is believed that antibodies that cross-compete with the exemplified antibody or bind to the same epitope as the exemplified antibody should also behave synergistically when treating humans having non-Hodgkin's lymphoma, chronic lymphocytic leukemia and/or acute lymphoblastic leukemia in combination with a purine analog, e.g., fludarabine.

An aspect of the present disclosure comprises a synergistic combination wherein the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine. In preferred aspects, the combination is used for the treatment of non-Hodgkin's lymphoma, chronic lymphocytic leukemia and/or acute lymphoblastic leukemia.

**Description of Drawings**

**Figure 1** shows the cytotoxicity effects of MOR208 and fludarabine alone and in combination on MEC-1 cells.

**Figure 2** shows the ADCC dose reponse curves of the combination of MOR208 and fludarabine in MEC-1 cells.

**Figure 3** shows the amino acid sequence of the variable domains of MOR208.

**Figure 4** shows the amino acid sequence of the Fc regions of MOR208.

**Figure 5** shows the normalized specific killing of MEC-1 target cells pretreated with Fludarabine (Flu) for 72 h. The data represents a pool of 3 independent experiments with 3 different effector cell donors.

**Figure 6** shows the mean tumor growth of the MOR208, FLU, and combination (MOR208/FLU) groups of the SCID mouse model described in Example 2.

**Figure 7** shows median survival time of the MOR208, FLU, and combination (MOR208/FLU) groups of the SCID mouse model described in Example 3.

**Detailed description of the invention**

"Synergy", "synergism" or "synergistic" mean more than the expected additive effect of a combination. The "synergy", "synergism" or "synergistic" effect of a combination is determined herein by the methods of Chou et al., Clarke et al., and/or Webb et al. See Ting-Chao Chou, Theoretical Basis, Experimental Design, and Computerized Simulation of Synergism and Antagonism in Drug Combination Studies, Pharmacol Rev 58:621-681 (2006). See also Clarke et al., Issues in experimental design and endpoint analysis in the study of experimental cytotoxic agents in vivo in breast cancer and other models, Breast Cancer Research and Treatment 46:255-278 (1997). See also Webb, J. L. (1963) Enzyme and Metabolic Inhibitors, Academic Press, New York.

The term "antibody" means monoclonal antibodies, including any isotype, such as, IgG, IgM, IgA, IgD and IgE. An IgG antibody is comprised of two identical heavy chains and two identical light chains that are joined by disulfide bonds. Each heavy and light chain contains a constant region and a variable region. Each variable region contains three segments called "complementarity-determining regions" ("CDRs") or "hypervariable regions", which are primarily responsible for binding an epitope of an antigen. They are referred to as CDR1, CDR2, and CDR3, numbered sequentially from the N-terminus. The more highly conserved portions of the variable regions outside of the CDRs are called the "framework regions". An "antibody fragment" means an Fv, scFv, dsFv, Fab, Fab' F(ab')2 fragment, or other fragment, which contains at least one variable heavy or variable light chain, each containing CDRs and framework regions.

A purine analog is an antimetabolite, which mimics the structure of metabolic purines, thereby interfering with the synthesis of nucleic acids. Fludarabine, for example, may be incorporated into RNA and DNA by substituting for the purine nucleotides, adenine and guanine. Purine analogs inhibit growth of fast proliferating cells of an individual, e.g. cancer cells, bone marrow cells or cells present in the gastrointestinal tract. Purine analogs include mercaptopurine, azathioprine, thioguanine and fludarabine.

Mercaptopurine is used in the treatment of acute leukemias, lymphomas, rheumatoid arthritis, and inflammatory bowel disease, such as Crohn's Disease and ulcerative colitis, respectively. Mercaptopurine has the following structure:

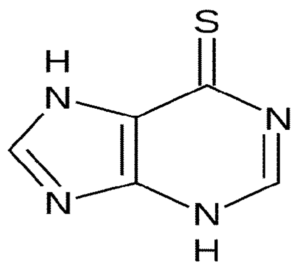

Azathioprine is the main immunosuppressive cytotoxic substance. It is widely used in transplantations to control rejection reactions. It is nonenzymatically cleaved to 6-mercaptopurine that acts as a purine analogue and an inhibitor of DNA synthesis. By preventing the clonal expansion of lymphocytes in the induction phase of the immune response, it affects both the cell and the humoral immunity. It also successfully suppresses autoimmunity. Azathioprine has the following structure:

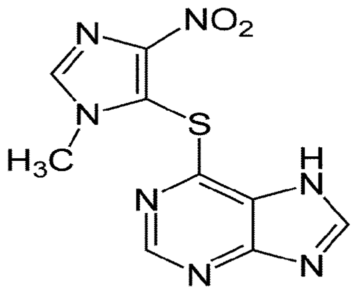

Thioguanine used during early and/or late intensification therapy of childhood acute lymphoblastic leukemia (ALL) while 6-mercaptopurine is mainly used at a different time point of therapy, namely during maintenance treatment of ALL. Thioguanine has the following structure:

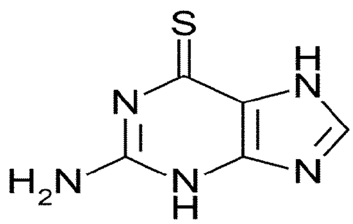

Fludarabine or fludarabine phosphate (Fludara®) is a chemotherapy drug used in the treatment of chronic lymphocytic leukemia and indolent non-Hodgkins lymphomas. Fludarabine is a purine analog. Fludarabine inhibits DNA synthesis by interfering with ribonucleotide reductase and DNA polymerase and and is S phase-specific (since these enzymes are highly active during DNA replication). Fludarabine has the following structure:

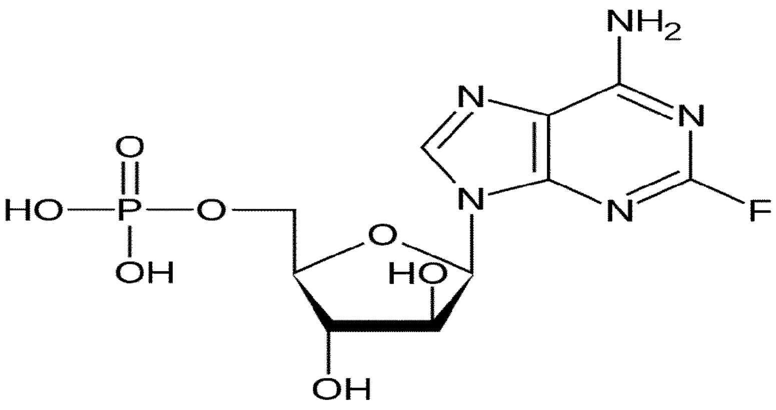

"FLU" when used herein means fludarabine.

"VH" refers to the variable region of an immunoglobulin heavy chain of an antibody, or antibody fragment. "VL" refers to the variable region of the immunoglobulin light chain of an antibody, or antibody fragment.

The term "CD19" refers to the protein known as CD19, having the following synonyms: B4, B-lymphocyte antigen CD19, B-lymphocyte surface antigen B4, CVID3, Differentiation antigen CD19, MGC12802, and T-cell surface antigen Leu-12.

Human CD19 has the amino acid sequence of:

MPPPRLLFFLLFLTPMEVRPEEPLVVKVEEGDNAVLQCLKGTSDGPTQQLTWSRESPLKPFLKLSL
GLPGLGIHMRPLAIWLFIFNVSQQMGGFYLCQPGPPSEKAWQPGWTVNVEGSGELFRWNVSDLG
GLGCGLKNRSSEGPSSPSGKLMSPKLYVWAKDRPEIWEGEPPCLPPRDSLNQSLSQDLTMAPGS
TLWLSCGVPPDSVSRGPLSWTHVHPKGPKSLLSLELKDDRPARDMWVMETGLLLPRATAQDAGK
YYCHRGNLTMSFHLEITARPVLWHWLLRTGGWKVSAVTLAYLIFCLCSLVGILHLQRALVLRRKRK
RMTDPTRRFFKVTPPPGSGPQNQYGNVLSLPTPTSGLGRAQRWAAGLGGTAPSYGNPSSDVQA
DGALGSRSPPGVGPEEEEGEGYEEPDSEEDSEFYENDSNLGQDQLSQDGSGYENPEDEPLGPE
DEDSFSNAESYENEDEELTQPVARTMDFLSPHGSAWDPSREATSLGSQSYEDMRGILYAAPQLR
SIRGQPGPNHEEDADSYENMDNPDGPDPAWGGGGRMGTWSTR. (SEQ ID NO: 7)

"MOR208" is an anti-CD19 antibody. The amino acid sequence of the variable domains is provided in Figure 3. The amino acid sequence of the heavy and light chain Fc regions of MOR208 is provided in Figure 4. "MOR208" and "XmAb 5574" are used as synonyms to describe the antibody shown in Figures 3 and 4. The MOR208 antibody is described in US patent application serial number 12/377,251.

Additional antibodies specific for CD19 are described in US patent no. 7,109,304 (Immunomedics); US application serial no. 11/917,750 (Medarex); US application serial no. 11/852,106 (Medimmune); US application serial no. 11/648,505 (Merck Patent GmbH); US patent no. 7,968,687 (Seattle Genetics); and US application serial no. 12/710,442 (Glenmark Pharmaceuticals).

"Fc region" means the constant region of an antibody, which in humans may be of the IgG1, 2, 3, 4 subclass or others. The sequences of human Fc regions are available at IMGT, Human IGH C-REGIONs, http://www.imgt.org/IMGTrepertoire/Proteins /protein/human/IGH/IGHC/Hu_IGHCallgenes.html (retrieved on 16 May 2011).

"RefmAb33" is an antibody whose amino acid sequence is as follows:

Heavy chain including the Fc region:

QVTLRESGPALVKPTQTLTLTCTFSGFSLSTAGMSVGWIRQPPGKALEWLADIWWDDKKH
YNPSLKDRLTISKDTSKNQVVLKVTNMDPADTATYYCARDMIFNFYFDVWGQGTTVTVSSASTKG
PSVFPLAPSSKSTSGGTAALGCLVKDYFPEPVTVSWNSGALTSGVHTFPAVLQSSGLYSLSSVVTV
PSSSLGTQTYICNVNHKPSNTKVDKKVEPKSCDKTHTCPPCPAPELLGGPDVFLFPPKPKDTLMIS
RTPEVTCVVVDVSHEDPEVQFNWYVDGVEVHNAKTKPREEQFNSTFRVVSVLTVVHQDWLNGKE
YKCKVSNKALPAPEEKTISKTKGQPREPQVYTLPPSREEMTKNQVSLTCLVKGFYPSDIAVEWESN
GQPENNYKTTPPMLDSDGSFFLYSKLTVDKSRWQQGNVFSCSVMHEALHNHYTQKSLSLSPGK
(SEQ ID NO: 8)

Light chain including the Fc region:

DIQMTQSPSTLSASVGDRVTITCSASSRVGYMHWYQQKPGKAPKLLIYDTSKLASGVPSRF
SGSGSGTEFTLTISSLQPDDFATYYCFQGSGYPFTFGGGTKVEIKRTVAAPSVFIFPPSDEQLKSGT
ASVVCLLNNFYPREAKVQWKVDNALQSGNSQESVTEQDSKDSTYSLSSTLTLSKADYEKHKVYAC
EVTHQGLSSPVTKSFNRGEC (SEQ ID NO: 9)

RefmAb33 is specific for RSV, and is used as isotype control, as it shares the same Fc region as MOR208.

A "combination" means more than one item, e.g. a compound such as an antibody and fludarabine.

The present disclosure also relates to combinations, pharmaceuticals, and pharmaceutical compositions containing the described combinations. The two components of the synergistic combination of the present invention, e.g. the antibody specific for CD19 and fludarabine, may be administered together, simultaneously or separately. When administered together, the two components may be formulated together in one pharmaceutical composition, which may include a pharmaceutical acceptable carrier or excipient. Alternatively the two components might also be formulated in

different pharmaceutical compositions. In this case the two components can be administered simultaneously or subsequently. In an embodiment, fludarabine, is administered prior to and/or separately from the administration of the antibody specific for CD19, e.g. MOR208.

A pharmaceutical composition includes an active agent, eg. an antibody for therapeutic use in humans. A pharmaceutical composition may include acceptable carriers or excipients.

"Administered" or "administration" includes but is not limited to delivery by an injectable form, such as, for example, an intravenous, intramuscular, intradermal or subcutaneous route or mucosal route, for example, as a nasal spray or aerosol for inhalation or as an ingestable solution, capsule or tablet.

A "therapeutically effective amount" of a compound or combination refers to an amount sufficient to cure, alleviate or partially arrest the clinical manifestations of a given disease or disorder and its complications. The amount that is effective for a particular therapeutic purpose will depend on the severity of the disease or injury as well as on the weight and general state of the subject. It will be understood that determination of an appropriate dosage may be achieved, using routine experimentation, by constructing a matrix of values and testing different points in the matrix, all of which is within the ordinary skills of a trained physician or clinical scientist.

The "CDRs" herein are defined by either Chothia et al or Kabat et al. See Chothia C, Lesk AM. (1987) Canonical structures for the hypervariable regions of immunoglobulins. J Mol Biol., 196(4):901-17. See Kabat E.A, Wu T.T., Perry H.M., Gottesman K.S. and Foeller C. (1991). Sequences of Proteins of Immunological Interest. 5th edit., NIH Publication no. 91-3242, US Dept. of Health and Human Services, Washington, DC.

"Cross competes" means the ability of an antibody or other binding agent to interfere with the binding of other antibodies or binding agents to CD19 in a standard competitive binding assay. The ability or extent to which an antibody or other binding agent is able to interfere with the binding of another antibody or binding molecule to CD19, and, therefore whether it can be said to cross-compete according to the invention, can be determined using standard competition binding assays. One suitable assay involves the use of the Biacore technology (e.g. by using the BIAcore 3000 instrument (Biacore, Uppsala, Sweden)), which can measure the extent of interactions using surface plasmon resonance technology. Another assay for measuring cross-competing uses an ELISA-based approach. A high throughput process for "epitope binning" antibodies based upon their cross-competition is described in International Patent Application No. WO 2003/48731

The term "epitope" includes any protein determinant capable of specific binding to an antibody or otherwise interacting with a molecule. Epitopic determinants generally consist of chemically active surface groupings of molecules such as amino acids or carbohydrate or sugar side chains and can have specific three-dimensional structural characteristics, as well as specific charge characteristics. An epitope may be "linear" or "conformational." The term "linear epitope" refers to an epitope with all of the points of interaction between the protein and the interacting molecule (such as an antibody) occur linearally along the primary amino acid sequence of the protein (continuous). The term "conformational epitope" refers to an epitope in which discontinuous amino acids that come together in three dimensional conformation. In a conformational epitope, the points of interaction occur across amino acid residues on the protein that are separated from one another.

"Binds the same epitope as" means the ability of an antibody or other binding agent to bind to CD19 and having the same epitope as the exemplified antibody. The epitopes of the exemplified antibody and other antibodies to CD19 can be determined using standard epitope mapping techniques. Epitope mapping techniques, well known in the art. include Epitope Mapping Protocols in Methods in Molecular Biology, Vol. 66 (Glenn E.Morris, Ed., 1996) Humana Press, Totowa, New Jersey. For example, linear epitopes may be determined by e.g., concurrently synthesizing large numbers of peptides on solid supports, the peptides corresponding to portions of the protein molecule, and reacting the peptides with antibodies while the peptides are still attached to the supports. Such techniques are known in the art and described in, e.g., U.S. Patent No. 4,708,871 ; Geysen et al, (1984) Proc. Natl. Acad. Sci. USA 8:3998-4002; Geysen et al, (1985) Proc. Natl. Acad. Sci. USA 82:78-182; Geysen et al, (1986) Mol. Immunol. 23 :709-715. Similarly, conformational epitopes are readily identified by determining spatial conformation of amino acids such as by, e.g., hydrogen/deuterium exchange, x-ray crystallography and two-dimensional nuclear magnetic resonance. See, e.g., Epitope Mapping Protocols, supra. Antigenic regions of proteins can also be identified using standard antigenicity and hydropathy plots, such as those calculated using, e.g., the Omiga version 1.0 software program available from the Oxford Molecular Group. This computer program employs the Hopp/Woods method, Hopp et al, (1981) Proc. Natl. Acad. Sci

USA 78:3824-3828; for determining antigenicity profiles, and the Kyte-Doolittle technique, Kyte et al, (1982) J.Mol. Biol. 157: 105-132; for hydropathy plots.

An aspect of the present disclosure comprises a combination of an antibody specific for CD19 and a purine analog for use in the treatment of non-Hodgkin's lymphoma, chronic lymphocytic leukemia and/or acute lymphoblastic leukemia. In embodiments, the combination is synergistic.

Herein, the combination of the exemplified anti-CD19 antibody and fludarabine behaved synergistically in *in vitro* and *in vivo* models relevant to NHL and CLL. As both NHL and CLL are B cell related disorders and CD19 is highly expressed on B-cells, the exemplified combination should have the same mechanism of action and should also behave

synergistically in the treatment of other B cell related disorders, e.g. ALL. Therefore, the combination of the exemplified antibody specific for CD19 and fludarabine will be effective in the treatment of humans in non-Hodgkin's lymphoma, chronic lymphocytic leukemia and/or acute lymphoblastic leukemia.

As the mechanism of action of fludarabine and other purine analogs are similar, in that purine analogs interfere with the synthesis of nucleic acids, it is believed that synergy should also be seen when treating humans having non-Hodgkin's lymphoma, chronic lymphocytic leukemia and/or acute lymphoblastic leukemia with a combination of the exemplified anti-CD19 antibody and a purine analog other than fludarabine, e.g., mercaptopurine, azathioprine, and thioguanine.

As the exemplified anti-CD19 antibody and other anti-CD19 antibodies bind to the CD19 antigen, it is believed that synergy should also be seen when treating humans having non-Hodgkin's lymphoma, chronic lymphocytic leukemia and/or acute lymphoblastic leukemia with a combination of any anti-CD19 antibody and a purine analog, where the anti-CD19 antibody is, for example, described in US patent application serial number 12/377,251 (Xencor), WO2005012493, WO2010053716 (Immunomedics); WO2007002223 (Medarex); WO2008022152 (Xencor); WO2008031056 (Medimmune); WO 2007/076950 (Merck Patent GmbH); WO 2009/052431 (Seattle Genetics); and WO2010095031 (Glenmark Pharmaceuticals)

As disclosed herein, hi embodiments, the antibody specific for CD19 comprises an antibody that cross-competes with the antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

As disclosed herein, the antibody specific for CD19 comprises an antibody that binds to the same epitope as an antibody comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6).

As disclosed herein, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6)..

In embodiments, the antibody specific for CD19 comprises a variable heavy chain of the sequence EVQLVESGGGLVKPGGSLKLSCAASGYTFTSYVMHWVRQAPGKGLEWIGYINPY NDGTKYNEKFQGRVTISSDKSISTAYMELSSLRSEDTAMYYCARGTYYYGTRVFDYWG QGTLVTVSS (SEQ ID NO: 10) and a variable light chain of the sequence DIVMTQSPATLSLSPGERATLSCRSSKSLQNVNGNTYLYWFQQKPGQSPQLLIYR MSNLNSGVPDRFSGSGSGTEFTLTISSLEPEDFAVYYCMQHLEYPITFGAGTKLEIK (SEQ ID NO: 11).

In embodiments, the antibody specific for CD19 comprises a heavy chain constant domain of the sequence ASTKGPSVFPLAPSSKSTSGGTAALGCLVKDYFPEPVTVSWNSGALTSGVHTFPAVLQSSGLYSL SSVVTVPSSSLGTQTYICNVNHKPSNTKVDKKVEPKSCDKTHTCPPCPAPELLGGPDVFLFPPKPK DTLMISRTPEVTCVVVDVSHEDPEVQFNWYVDGVEVHNAKTKPREEQFNSTFRVVSVLTVVHQD WLNGKEYKCKVSNKALPAPEEKTISKTKGQPREPQVYTLPPSREEMTKNQVSLTCLVKGFYPSDIA VEWESNGQPENNYKTTPPMLDSDGSFFLYSKLTVDKSRWQQGNVFSCSVMHEALHNHYTQKSL SLSPGK. (SEQ ID NO: 12)

In embodiments, the antibody specific for CD19 comprises a light chain constant domain of the sequence RTVAAPSVFIFPPSDEQLKSGTASVVCLLNNFYPREAKVQWKVDNALQSGNSQESVTEQDSKD STYSLSSTLTLSKADYEKHKVYACEVTHQGLSSPVTKSFNRGEC (SEQ ID NO: 13).

In embodiments, the purine analog is fludarabine.

In embodiments, the components of the combination, the antibody specific for CD19 and fludarabine, are administered separately. In an embodiment, fludarabine is administered prior to administration of the antibody specific for CD19.

As disclosed herein, the combination is a pharmaceutical composition. As disclosed herein, the composition comprises an acceptable carrier. As disclosed herein, the combination is administered in an effective amount.

In an aspect the present disclosure comprises the synergistic combination of an antibody specific for CD19 comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine is able to mediate killing of MEC-1 cells by ADCC in the presence of isolated human PBMCs with an at least two-fold, three-fold, four-fold, or five-fold better efficacy than fludarabine alone.

An aspect of the present disclosure comprises a synergistic combination of an antibody specific for CD19

comprising an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6) and fludarabine for the treatment of non-Hodgkin's lymphoma, chronic lymphocytic leukemia and/or acute lymphoblastic leukemia. In embodiments, the non-Hodgkin's lymphoma is selected from the group consisting of follicular lymphoma, small lymphocytic lymphoma, mucosa-associated lymphoid tissue, marginal zone, diffuse large B cell, Burkitt's, and mantle cell.

Another aspect of the present disclosure comprises a method of treating non-Hodgkin's lymphoma, chronic lymphocytic leukemia and/or acute lymphoblastic leukemia in an individual in need thereof, which method comprises administration of an antibody specific for CD19 and a purine analog. In an aspect of the present disclosure, the antibody specific for CD19 comprises an HCDR1 region of sequence SYVMH (SEQ ID NO: 1), an HCDR2 region of sequence NPYNDG (SEQ ID NO: 2), an HCDR3 region of sequence GTYYYGTRVFDY (SEQ ID NO: 3), an LCDR1 region of sequence RSSKSLQNVNGNTYLY (SEQ ID NO: 4), an LCDR2 region of sequence RMSNLNS (SEQ ID NO: 5), and an LCDR3 region of sequence MQHLEYPIT (SEQ ID NO: 6). In embodiments of the method, the purine analog is fludarabine.

**Examples**

Example 1: Inhibition of proliferation of MEC-1 cells using MOR208 and fludarabine alone and in combination

Materials

MEC-1 cells: chronic β-cell leukemia cell line DSMZ# ACC497; Cell Medium: Iscove's Modified Dulbecco's Medium (IMDM) with GlutaMAX™, Invitrogen, Cat No.: 31980-048, 20% FCS; PBMCs: RPMI1640, with stabile Glutamine, PAN Biotech GmbH, Cat No.: P04-13500 supplemented with 10% FCS; Biocoll: Biochrome AG CAT No.: L6115 LOT No.: 1050T; Fludarabine: Bayer, 25 mg/ml in ddH2O, LOT No.: 91 00ST; and RefmAb33 (anti-RSV) with same Fc region as MOR208.

Methods

The cytotoxicity of MOR208 and fludarabine alone and in combination was tested in MEC-1 cells. FLU is a purine analog, therefore, functions via direct cytoxicity in MEC-1 cells. MOR208 targets CD19 and additionally functions via ADCC in killing MEC-1 cells. For the following groups MEC-1 cell killing was measured: FLU at 18μg/ml; MOR208 at 66pm and the combination of MOR208 at 66pm and FLU at 18μg/ml. These concentrations were chosen as they are near or at the EC50 for MOR208 and FLU.

In the FLU group and MOR208+FLU combination group, the MEC-1 cells were pre-incubated with FLU for 72 hours prior to the ADCC assay measurements. The MEC-1 cells were stained using 1 mg/ml Calcein AM then counted and adjusted to $2\times10^5$/ml. The PBMCs were counted and adjusted to $6\times10^6$/ml. The ADCC assays were done as follows: Using 96 well plates, a 100μl cell suspension of MEC-1 cells was added per well, then 100μl cell suspension of PBMCs was added to each well resulting in an E:T ratio of 30:1. The antibodies were diluted to 1pg/ml in medium. Cells were centrifuged and re-suspended. To the target:effector cell-pellet 100μl antibody solution or according control solution was added. The mixture was incubated for 4h in CO2-incubator at 37°C. The ADCC measurements were taken as follows: the incubated cell solution (~100μl) was transfered into FACS tubes and 200μl FACS buffer (DPBS + 3%FCS) and 0,5 μ! PI stock solution was added to each tube. FACS-Calibur was used. Dead MEC-1 cells were stained with propidium iodide.

Table 1 and Figure 1 show the raw data.

Table 1

| | control | MOR208 66pm | FLU 18μg/ml | FLU+MOR208 combination |
|---|---|---|---|---|
| Experiment 1 | 11 | 35,2 | 36,39 | 52,14 |
| Experiment 2 | 19,5 | 29,8 | 38,48 | 46,9 |
| Experiment 3 | 29,9 | 47,01 | 57,27 | 65,63 |

The values represent % dead cells. Each experiment represents PBMCs from different donors. The contol used for Experiments 1 and 2 was RefMab33. The control used for Experiment 3, was PBMCs alone.

Table 2 shows the raw data of Table 1 normalized for specific killing and the results of the Chou calculations

done in the determination of synergism.

Table 2

| | Fludarabine 18 μg/ml | Mor208 66 pM | Flu + MOR208 (combination) | Chou Index |
|---|---|---|---|---|
| **Experiment 1** | 0,6 | 0,6 | 1,0 | 0,03 |
| **Experiment 2** | 0,7 | 0,4 | 1,0 | 0,3 |
| **Experiment 3** | 0,8 | 0,5 | 1,0 | 0,3 |
| **Average** | 0,7 | 0,5 | 1,0 | 0,2 |

The values shown in Table 2 are calculated as follows: 1) from the raw data (% dead cells) shown in Table 1, the background (controls) were subtracted, resulting in the specific killing for each treatment group; then 2) the specific killing values were normalized by setting the combination of MOR208 + FLU to 1. The averages of Table 2 are depicted in Figure 5. Example ADCC dose response curves used in the Chou factor calculations of the MOR208 + FLU combination are shown in Figure 2.

Chou Index (CI) calculations were completed in order to determine synergy of the combination of the exemplified anti-CD19 antibody and fludarabine as compared to MOR208 and FLU alone. The calculations are described in Ting-Chao Chou, Theoretical Basis, Experimental Design, and Computerized Simulation of Synergism and Antagonism in Drug Combination Studies, Pharmacol Rev 58:621-681 (2006)and Chou TC, Talalay P, Quantitative analysis of dose-effect relationships: the combined effects of multiple drugs or enzyme inhibitors. Adv Enzyme Regul 22: 27-55 (1984). The methods of Chou-Talalay are carried out using the CI-isobol method.

Median-effect equation

The median-effect equation models of the effect of an inhibitor (such as a drug) as $F_a/F_u$ =(D/D50)^m, where D is the dose, $F_a$ and $F_u$ is the fraction of the system affected and unaffected by the dose D ($F_a + F_u = 1$); D50 is the dose producing the median effect (e.g. IC50, ED50, LD50). The constant m determines the shape of the dose-effect curve. We used Excel Fit software to carry out a linear regression calculation to estimate the parameters m and D50.

The effects of the combination on MEC-1 cells is measured % cell death as described above. We define the fraction $F_u$ to be the ratio of % cell death of the treated cell line to the % cell death of the cell line exposed to a control. That is:

$$F_u = \text{\% cell death(treated cell line)/ \% cell death (non-treated cell line)}$$

Then the % cell death of a cell line is the constant D50 in the median effect equation, which can be estimated by the linear regression described above.

CI-isobol method

The CI-isobol method provides a quantitative assessment of synergism between drugs. A combination index (CI) is estimated from dose-effect data of single and combined drug treatments. A value of CI less than 1 indicates synergism; CI = 1 indicates additive effect; and CI > 1 indicates antagonism. Drug interaction (synergism or antagonism) is more pronounced the farther a CI value is from 1.

Formally, the combination index (CI) of a combined drug treatment is defined as $CI = D_1/D_{x1} + D_2/D_{X2}$. Here D1 and D2 are the doses of drug 1 and drug 2 of the combination, respectively; and Dx1, and Dx2 is the dose of a treatment with only drug 1 and drug 2 that would give the same effect as that of the combination. The doses Dx1 and Dx2 need to be estimated from the dose-effect data of single drug treatments. Essentially, a median effect equation is fitted to the data of each drug. From the median effect equation of a drug, we can estimate the dose (i.e. D) necessary to produce an effect (i.e. Fa, Fu). The further a point lies from the additive line, the bigger the different between 1 and its CI, thus the stronger the (synergistic or antagonistic) effect is.

Results

As shown in Table 2, the Chou index values indicate clear synergism of the combination of MOR208 and fludarabine in the specific killing of MEC-1 cells as compared to MOR208 and fludarabine alone. This conclusion is based upon the Chou calculations of 0.03, 0.3 and 0.3 in each of the three experiments, respectively, with an average

of 0.21, where a CI <1 indicates synergism. Therefore, the combination of MOR208 and fludarabine will also behave synergistically in the treatment of non-Hodgkin's lymphoma (NHL), chronic lymphoid leukemia (CLL), and/or acute lymphoblastic leukemia (ALL) in humans.

In order to confirm the results of the above Chou calculations, the normalized data of Table 2 was evaluated for statistical significance using the Bonferroni's Multiple Comparison Test. See James, et al, Antibody-mediated B-cell depletion before adoptive immunotherapy with T cells expressing CD20-specific chimeric T-cell receptors facilitates eradication of leukemia in immunocompetent mice, Blood, 114(27):5454-63 (Epub 2009 Oct 30). The results are shown in Table 3.

Table 3

| Bonferroni's Multiple Comparison Test | Mean Diff. | T value | Significant? (P < 0.05) | Summary |
|---|---|---|---|---|
| Fludarabine (18μg/ml) vs. FLU + MOR 208 combination | -0.3067 | 5.039 | Yes | ** |
| MOR208 (66pM) vs. FLU + MOR208 combination | -0.5167 | 8.490 | Yes | *** |
| ** p < 0,01<br>*** p < 0,001 | | | | |

Results

As shown in Table 3, the Bonferroni's Multiple Comparison Test shows that the combination treatment of FLU + MOR208 is statistically more effective in the specific killing of MEC-1 cells than the treatment of FLU and MOR208 alone.

Example 2: MOR208 and FLU alone and in combination in subcutaneously (SC)-implanted human Ramos Burkitt's β-cell lymphoma tumor growth model.

Materials

RAMOS human Burkitt's lymphoma cells (ATCC number CRL-1596, lot# 3953138); Vehicle control: 0.9% sodium chloride, 25mg/ml mannitol, pH 7.0;; SCID Mice (University of Adelaide, Waite Campus, Urrbaraie, SA, Australia, Strain C.B.-17-Igh-$1^b$-$Prkdc^{scid}$).

Methods

Six-to-seven-week old female C.B-17 SCID mice were implanted sub-cutaneously with RAMOS cells (~5 × $10^6$ cells/mouse). 14 days after inoculation, the mice were separated into ten groups of eight, where each group had tumor volumes of relatively the same size. Treatment began on Day 14. The treatment regimens are provided in Table 4. The study duration was 63 days.

Table 4

| No. of Animals | Test Articles | Dose (mg/kg) | Treatment Route and Schedule |
|---|---|---|---|
| 8 | Fludarabine | 125 | IP, Q1Dx5 |
| 8 | Fludarabine | 250 | IP, Q1Dx5 |
| 8 | MOR208 | 1* -> 10 | Dys 14*, 17*, 21*, 24, 28, 31, 35, and 38 |
| 8 | Vehicle | | IP, Q1Dx5 |
| 8 | MOR208/ Fludarabine | 1*->10/ 125 | Dys 14*, 17*, 21*, 24, 28, 31, 35, and 38/ IP, Q1Dx5 |

MOR208, fludarabine, and the vehicle were administered in a volume of 0.1 mL/10 g of body weight. The initial readout was tumor growth, specifically tumor volume at study day 38. Tumor weights were calculated using the equation (l × w2)/2, where l and w refer to the larger and smaller dimensions collected at each measurement.

The results are shown in Table 5 and the averages are depicted in Figure 6.

Table 5

| Tumor Volume [mm^3] at study day 38 | | | | | |
|---|---|---|---|---|---|
| | Vehicle Control | MOR208 10 mg/kg | Fludarabine 125 mg/kg | Fludarabine 250 mg/kg | Combination: MOR208/FLU 125 |
| Group 1 | 2890 | 2138 | 1666 | 1352 | 1268 |
| Group 2 | 4400 | 2025 | 2560 | 1352 | 750 |
| Group 3 | 4200 | 3179 | 864 | 2816 | 726 |
| Group 4 | 4152 | 1764 | 1913 | 1764 | 446 |
| Group 5 | 3791 | 1862 | 3564 | 650 | 936 |
| Group 6 | 4513 | 3402 | 2560 | 787 | 1268 |
| Group 7 | 4152 | 2560 | 2025 | 1800 | 787 |
| Group 8 | | 2816 | 1437 | 787 | |
| **Average** | **4014** | **2468** | **2073** | **1413** | **883** |

In addition to an endpoint of tumor volume at study day 38, the parameters of 1) reduced tumor growth [%] at day 38, and 2) increased time to 4000mg tumors [%] were evaluated. The results are shown in Table 6.

Table 6

| Treatment Groups | Reduced tumor growth at study Day 38 (%) | Increased time to 4000mg [%] |
|---|---|---|
| MOR208 | 38.5 | 17.52 |
| FLU 125 | 48.3 | 22.37 |
| FLU 250 | 64.8 | 30.19 |
| Combination of MOR208 and FLU 125 | 78 | 59.3 |
| Control | 0 | 0 |

Both of the endpoints shown in Table 6 were evaluated using the Fractional Product Concept (FPC) in order to determine if synergism existed with the treatment of the combination of MOR208 and FLU treatment as compared to MOR208 and Fludarabine alone. When the combination treatment group is more effective than the FPC calculation, then synergism exists. The Fractional Product Concept was calculated using the formula 1-[(1-A)*(1-B)] = fpc(%), as described by Webb, J. L. (1963) Enzyme and Metabolic Inhibitors, Academic Press, New York. After the FPC calculations were completed, the resulting FPC value and the values from Table 6 were normalized by setting the FPC value to 1. The results of these calculations are shown in Table 7.

Table 7

| | MOR208 [10 mg/kg] | Fludarabine [125 mg/kg] | Fludarabine [250 mg/kg] | Fractional Product combination | MOR208 + FLU 125 combination | Effect |
|---|---|---|---|---|---|---|
| Reduced tumor growth at study Day 38 (%) | 0.57 | 0.75 | 0,9 | 1 | 1.15 | Synergism |
| Increased time to 4000mg [%] | 0.48 | 0.62 | 0,8 | 1 | 1.65 | Synergism |

Results

Both of the endpoints are a measure of inhibition of tumor growth and in both endpoints, reduced tumor growth (%) at study day 38 and increased time (%) to 4000mg, the combination of MOR208 + FLU125 showed clear synergism in comparision to MOR208 and FLU alone.

In order to confirm the results of the above FPC calculations, the data of average Tumor Volume [mm^3] at study day 38 (shown in Table 5) was evaluated for statistical significance using the Bonferroni's Multiple Comparison Test. The results are shown in Table 8.

Table 8

| Treatment Group | Bonferroni's Multiple Comparison Test; p value |
|---|---|
| FLU125 vs FLU125 + MOR208 (combination) | $p < 0.01$ |
| FLU250 vs FLU 125 + MOR208 (combination) | n.s. |
| MOR208 vs FLU125 + MOR208 (combination) | $p < 0.001$ |
| Control vs. FLU 125 | $p < 0.001$ |
| Control vs. FLU250 | $p < 0.001$ |
| Control vs. FLU125 + MOR208 (combination) | $p < 0.001$ |
| Control vs. MOR208 | $p < 0.001$ |
| A p value of < 0.05 shows statistical significance. | |

Results

As shown in Tables 5 and 6, in all parameters, the combination of MOR208 and Fludarabine 125 mg/kg was more effective in the inhibition of tumor growth *in vivo* than MOR208 or Fludarabine alone. This increase in effectiveness of the combination of MOR208 and Fludarabine 125 mg/kg in the inhibition of tumor growth *in vivo* as compared to both MOR208 and Fludarabine alone was confirmed to be statistically significant, as shown in Table 8. In addition, the Fractional Product Concept calculations show clear synergism of combination of MOR208 and Fludarabine 125 mg/kg in the inhibition of tumor growth *in vivo* as compared to MOR208 or Fludarabine alone, as shown in Table 7. Therefore, the combination of MOR208 and fludarabine will also behave synergistically in the treatment of non-Hodgkin's lymphoma (NHL), chronic lymphoid leukemia (CLL), and acute lymphoblastic leukemia (ALL) in humans.

In addition, the combination of MOR208 and Fludarabine 125 mg/kg was more effective in the inhibition of tumor growth *in vivo* than the higher dose Fludarabine 250 mg/kg alone. As fludarabine has shown significant side effects at higher doses, this result shows that a safer, more effective alternative to high dose of Fludarabine, is the combination of MOR208 and Fludarabine.

Example 3 MOR208 and fludarabine alone and in combination in human Non-hodgkin RAMOS tumor in SCID mice, survival model

Materials

Cyclophosphamide (Fluka, Buchs Switzerland, Lot. No. 07551661); Vehicle Control: 0.9% sodium chloride, 25mg/ml mannitol, pH 7.0; SCID Mice (University of Adelaide, Waite Campus, Urrbaraie, SA, Australia, Strain C.B.-17-Igh-$1^b$-Prkdc$^{scid}$); RAMOS human lymphoma cells (ATCC number CRL-2638).

Methods

SCID mice were pre-treated with Cyclophosphamide (18 mg/kg, i.p., twice daily) for two days prior to RAMOS cell inoculation (Day -5 and -4). On the day of inoculation (Day -3), the mice were separated into ten groups of eight mice each, and inoculated with $1 \times 10^6$ RAMOS cells each intravenously into the tail vein. The dosing regimen for each group is shown in Table 9 and commenced on Day 0. The study duration was three weeks.

Table 9: Dosing regimen

| Group | Compound | Treatment | Intended Schedule |
|---|---|---|---|
| A | Fludarabine | 125 mg/kg, i.p, in 10 mL/kg | Once daily (Day 0-5) |
| B | MOR208 | 3 mg/kg, i.v., in 10 mL/kg | Twice weekly for 3 weeks |
| C | Vehicle Control | i.p., 10 mL/kg | Once daily (Day 0-5) |
| AB | Fludarabine /MOR208 | 125 mg/kg, i.p; 3 mg/kg, i.v. in 10 mL/kg; | Once daily (Day 0-5) /twice weekly for 3 weeks |
| E | Fludarabine | 250 mg/kg, i.p, in 10 mL/kg | Once daily (Day 0-5) |

The readout was median survival time in days. In order to evaluate whether the effect of the combination as compared to the individual treatment groups was synergistic, the methods of Clark et al. were used.

Clarke et al. synergism

The method is described in Issues in experimental design and endpoint analysis in the study of experimental cytotoxic agents *in vivo* in breast cancer and other models, Breast Cancer Research and Treatment 46:255-278 (1997).

The data is analysed in the following way:

Antagonistic $(AB)/C < (A/C) \times (B/C)$

Additive $(AB)/C = (A/C) \times (B/C)$

Synergistic $(AB)/C > (A/C) \times (B/C)$

where A is the treatment with MOR208; B is the treatment with FLU alone; C is the response to the treatment vehicle; AB is the combination of treatments A and B. The median survival time in days for each study group and the Clarke et al. analysis are shown in Table 10.

Table 10

| | Median Survival Time (Days) |
|---|---|
| A = response to treatment MOR208 | 23,5 |
| B = response to treatment FLU | 22 |
| C = response to treatment vehicle | 18,5 |
| AB = combination of treatments A and B | 31 |
| | |
| (AB)/C | 1,675675676 |
| | is bigger than |
| (A/C) × (B/C) | 1,510591673 |
| | = Synergism |

Results

The combination of MOR208 and FLU showed clear synergism in median survival days as compared to MOR208 and FLU alone.

In order to confirm the results of the above synergism calculations, the median survival time in days of the combination of MOR208 and fluradabine 125 mg/kg was compared to MOR208 and fluradabine alone using the Mantel-Haenszel test [P value], and Gehan-Wilcoxon test [P value]. Results shown in Table 11.

Table 11

| Treatment Group | Mantel-Haenszel test [P value] | Gehan-Wilcoxon test [P value] |
|---|---|---|
| FLU/MOR vs. FLU 125 | 0,0008 | 0,0012 |
| FLU/MOR vs. MOR | 0,0001 | 0,0004 |
| FLU/MOR vs. FLU250 | 0,0011 | 0,0016 |
| FLU/MOR vs. control | 0,0001 | 0,0004 |
| Control vs. FLU 125 | 0,0061 | 0,0162 |
| Control vs. FLU250 | 0,0061 | 0,0162 |
| Control vs. MOR208 | < 0.0001 | 0,0002 |

Results

Table 10 shows that the combination of MOR208 and Fludarabine 125 mg/kg synergistically increased the median survival time in the Burkitt's lymphoma SCID mouse model as compared to both MOR208 and Fludarabine alone. This increase of median survival time of the combination of MOR208 and Fludarabine 125 mg/kg *in vivo* was confirmed to be statistically significant as compared to both MOR208 and Fludarabine alone, as shown in Table 11. Therefore, the combination of MOR208 and fludarabine will also behave synergistically in the treatment of non-Hodgkin's lymphoma (NHL), chronic lymphoid leukemia (CLL), and acute lymphoblastic leukemia (ALL) in humans.

In addition, the combination of MOR208 and Fludarabine 125 mg/kg was more effective in increasing median survival time *in vivo* than the higher dose Fludarabine 250 mg/kg alone. As fludarabine has shown significant side effects at higher doses, despite its effectiveness, this result shows that a safer, more effective alternative to high dose Fludarabine, is the combination of MOR208 and Fludarabine.

## Claims

1. A synergistic combination of an antibody specific for CD19 and fludarabine for use in the treatment of non-Hodgkin's lymphoma, chronic lymphocytic leukemia or acute lymphoblastic leukemia, wherein the antibody comprises a variable heavy chain of the sequence EVQLVESGGGLVKPGGSLKLSCAASGYTFTSYVMHWVRQAPGKGLEWIGYINPYNDGTKYNEKFQGRVTISSDKSISTAYMELSSLRSEDTAMYYCARGTYYYGTRVFDYWGQGTLVTVSS (SEQ ID NO: 10) and a variable light chain of the sequence DIVMTQSPATLSLSPGERATLSCRSSKSLQNVNGNTYLYWFQQKPGQSPQLLIYRMSNLNSGVPDRFSGSGSGTEFTLTISSLEPEDFAVYYCMQHLEYPITFGAGTKLEIK (SEQ ID NO: 11) and a heavy chain constant domain of the sequenceASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPDVFLFPPKPKDTLMISRTPEVTCVVVDVSHEDPEVQFNWYVDGVEVHNAKTKPREEQFNSTFRVVSVLTVVHQDWLNGKEYKCKVSNKALPAPEEKTISKTKGQPREPQVYTLPPSREEMTKNQVSLTCLVKGFYPSDIAVEWESNGQPENNYKTTPPMLDSDGSFFLYSKLTVDKSRWQQGNVFSCSVMHEALHNHYTQKSLSLSPGK (SEQ ID NO: 12).

2. The combination for use according to claim 1, wherein the antibody comprises a light chain constant domain of the sequence RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC (SEQ ID NO: 13).

3. The combination according to any one of the preceding claims for use in the treatment of non-Hodgkin's lymphoma, optionally wherein the non-Hodgkin's lymphoma is selected from the group consisting of follicular lymphoma, small lymphocytic lymphoma, mucosa-associated lymphoid tissue lymphoma, marginal zone lymphoma, diffuse large B cell lymphoma, Burkitt's lymphoma, and mantle cell lymphoma.

4. The combination for use according to claim 3, wherein the non-Hodgkin's lymphoma is follicular lymphoma.

5. The combination for use according to claim 3, wherein the non-Hodgkin's lymphoma is small lymphocytic lymphoma.

6. The combination for use according to claim 3, wherein the non-Hodgkin's lymphoma is mucosa-associated lymphoid tissue lymphoma.

7. The combination for use according to claim 3, wherein the non-Hodgkin's lymphoma is marginal zone lymphoma.

8. The combination for use according to claim 3, wherein the non-Hodgkin's lymphoma is diffuse large B cell lymphoma.

9. The combination for use according to claim 3, wherein the non-Hodgkin's lymphoma is Burkitt's lymphoma.

10. The combination for use according to claim 3, wherein the non-Hodgkin's lymphoma is mantle cell lymphoma.

11. The combination according to claim 1 or claim 2 for use in the treatment of chronic lymphocytic leukemia.

12. The combination according to claim 1 or claim 2 for use in the treatment of acute lymphoblastic leukemia.

**13.** The combination for use according to any one of the preceding claims, wherein the antibody specific for CD19 and fludarabine are:

(a) formulated together in one pharmaceutical composition; or
(b) formulated in different pharmaceutical compositions.

**14.** The combination for use according to any one of claims 1 to 13, wherein said antibody specific for CD19 and fludarabine are administered simultaneously.

**15.** The combination for use according to any one of claims 1 to 12 or claim 13(b), wherein said antibody specific for CD19 and fludarabine are administered separately.

**16.** The combination for use according to claim 15, wherein fludarabine is administered prior to administration of said antibody specific for CD19.

**Patentansprüche**

**1.** Synergistische Kombination von einem für CD19 spezifischen Antikörper und Fludarabin zur Verwendung bei der Behandlung von Non-Hodgkin-Lymphom, chronischer lymphatischer Leukämie oder akuter lymphoblastischer Leukämie, wobei der Antikörper eine variable schwere Kette mit der

EVQLVESGGGLVKPGGSLKLSCAASGYTFTSYVMHWVRQAPGKGLEWIGYINPYNDG
TKYNEKFQGRVTISSDKSISTAYMELSSLRSEDTAMYYCARGTYYYGTRVFDYWGQG
TLVTVSS (SEQ ID NO: 10)

Sequenz
und eine variable leichte Kette mit der Sequenz

DIVMTQSPATLSLSPGERATLSCRSSKSLQNVNGNTYLYWFQQKPGQSPQLLIYRMSN
LNSGVPDRFSGSGSGTEFTLTISSLEPEDFAVYYCMQHLEYPITFGAGTKLEIK (SEQ ID
NO: 11)

und eine Schwere-Kette-konstante Domäne mit der Sequenz

ASTKGPSVFPLAPSSKSTSGGTAALGCLVKDYFPEPVTVSWNSGALTSGVHTFPAVLQ
SSGLYSLSSVVTVPSSSLGTQTYICNVNHKPSNTKVDKKVEPKSCDKTHTCPPCPAPE
LLGGPDVFLFPPKPKDTLMISRTPEVTCVVVDVSHEDPEVQFNWYVDGVEVHNAKTKP
REEQFNSTFRVVSVLTVVHQDWLNGKEYKCKVSNKALPAPEEKTISKTKGQPREPQV
YTLPPSREEMTKNQVSLTCLVKGFYPSDIAVEWESNGQPENNYKTTPPMLDSDGSFFL
YSKLTVDKSRWQQGNVFSCSVMHEALHNHYTQKSLSLSPGK (SEQ ID NO: 12)

umfasst.

**2.** Kombination zur Verwendung nach Anspruch 1, wobei der Antikörper eine Leichte-Kette-konstante Domäne mit der Sequenz

RTVAAPSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ ID NO: 13)

umfasst.

3. Kombination nach einem der vorhergehenden Ansprüche zur Verwendung bei der Behandlung von Non-Hodgkin-Lymphom, gegebenenfalls wobei das Non-Hodgkin-Lymphom ausgewählt ist aus der Gruppe bestehend aus follikulärem Lymphom, kleinzelligem lymphozytischem Lymphom, MALT(Mucosa-Associated Lymphoid Tissue)-Lymphom, Marginalzonen-Lymphom, diffusem großzelligem B-Zell-Lymphom, Burkitt-Lymphom und Mantelzell-Lymphom.

4. Kombination zur Verwendung nach Anspruch 3, wobei es sich bei dem Non-Hodgkin-Lymphom um follikuläres Lymphom handelt.

5. Kombination zur Verwendung nach Anspruch 3, wobei es sich bei dem Non-Hodgkin-Lymphom um kleinzelliges lymphozytisches Lymphom handelt.

6. Kombination zur Verwendung nach Anspruch 3, wobei es sich bei dem Non-Hodgkin-Lymphom um MALT-Lymphom handelt.

7. Kombination zur Verwendung nach Anspruch 3, wobei es sich bei dem Non-Hodgkin-Lymphom um Marginalzonen-Lymphom handelt.

8. Kombination zur Verwendung nach Anspruch 3, wobei es sich bei dem Non-Hodgkin-Lymphom um diffuses großzelliges B-Zell-Lymphom handelt.

9. Kombination zur Verwendung nach Anspruch 3, wobei es sich bei dem Non-Hodgkin-Lymphom um Burkitt-Lymphom handelt.

10. Kombination zur Verwendung nach Anspruch 3, wobei es sich bei dem Non-Hodgkin-Lymphom um Mantelzell-Lymphom handelt.

11. Kombination nach Anspruch 1 oder Anspruch 2 zur Verwendung bei der Behandlung von chronischer lymphatischer Leukämie.

12. Kombination nach Anspruch 1 oder Anspruch 2 zur Verwendung bei der Behandlung von akuter lymphoblastischer Leukämie.

13. Kombination zur Verwendung nach einem der vorhergehenden Ansprüche, wobei der für CD19 spezifische Antikörper und Fludarabin:

(a) zusammen in einer pharmazeutischen Zusammensetzung formuliert sind; oder
(b) in verschiedenen pharmazeutischen Zusammensetzungen formuliert sind.

14. Kombination zur Verwendung nach einem der Ansprüche 1 bis 13, wobei der für CD19 spezifische Antikörper und Fludarabin gleichzeitig verabreicht werden.

15. Kombination zur Verwendung nach einem der Ansprüche 1 bis 12 oder Anspruch 13(b), wobei der für CD19 spezifische Antikörper und Fludarabin getrennt verabreicht werden.

16. Kombination zur Verwendung nach Anspruch 15, wobei Fludarabin vor Verabreichung des für CD19 spezifischen Antikörpers verabreicht wird.

**Revendications**

1. Combinaison synergique d'un anticorps spécifique du CD19 et de fludarabine pour une utilisation dans le traitement d'un lymphome non Hodgkinien, d'une leucémie lymphocytaire chronique ou d'une leucémie lymphoblastique aiguë, dans laquelle l'anticorps comprend une chaîne lourde variable de la séquence

EVQLVESGGGLVKPGGSLKLSCAASGYTFTSYVMHVWVRQAPGKGLEWIG

YINPYNDGTKYNEKFQGRVTISSDKSISTAYMELSSLRSEDTAMYYCARGTYYYG TRVFDYWGQGTLVTVSS (SEQ ID NO: 10) et une chaîne légère variable ayant la séquence DIVMTQSPATLSLSPGERATLSCRSSKSLQNVNGNTYLYWFQQKPGQSPQ LLIYRMSNLNSGVPDRFSGSGSGTEFTLTISSLEPEDFAVYYCMQHLEYPITFGA GTKLEIK (SEQ ID NO: 11) et un domaine constant de chaîne lourde ayant la séquence

ASTKGPSVFPLAPSSKSTSGGTAALGCLVKDYFPEPVTVSWNSGALTSGV HTFPAVLQSSGLYSLSSVVTVPSSSLGTQTYICNVNHKPSNTKVDKKVEPKSCDK THTCPPCPAPELLGGPDVFLFPPKPKDTLMISRTPEVTCVVVDVSHEDPEVQFNW YVDGVEVHNAKTKPREEQFNSTFRVVSVLTVVHQDWLNGKEYKCKVSNKALPAPE EKTISKTKGQPREPQVYTLPPSREEMTKNQVSLTCLVKGFYPSDIAVEWESNGQP ENNYKTTPPMLDSDGSFFLYSKLTVDKSRWQQGNVFSCSVMHEALHNHYTQKSLS LSPGK (SEQ ID NO: 12).

**2.** Combinaison pour une utilisation selon la revendication 1, dans laquelle l'anticorps comprend un domaine constant de chaîne légère de la séquence RTVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTKSFNRG EC (SEQ ID NO: 13).

**3.** Combinaison selon l'une quelconque des revendications précédentes pour une utilisation dans le traitement d'un lymphome non Hodgkinien, éventuellement dans laquelle le lymphome non Hodgkinien est choisi dans le groupe constitué par un lymphome folliculaire, un petit lymphome lymphocytaire, un lymphome du tissu lymphoïde associé aux muqueuses, un lymphome de la zone marginale, un lymphome diffus à grands lymphocytes B, un lymphome de Burkitt et un lymphome à cellules du manteau.

**4.** Combinaison pour une utilisation selon la revendication 3, dans laquelle le lymphome non Hodgkinien est un lymphome folliculaire.

**5.** Combinaison pour une utilisation selon la revendication 3, dans laquelle le lymphome non Hodgkinien est un petit lymphome lymphocytaire.

**6.** Combinaison pour une utilisation selon la revendication 3, dans laquelle le lymphome non Hodgkinien est un lymphome du tissu lymphoïde associé aux muqueuses.

**7.** Combinaison pour une utilisation selon la revendication 3, dans laquelle le lymphome non Hodgkinien est un lymphome de la zone marginale.

**8.** Combinaison pour une utilisation selon la revendication 3, dans laquelle le lymphome non Hodgkinien est un lymphome diffus à grands lymphocytes B.

**9.** Combinaison pour une utilisation selon la revendication 3, dans laquelle le lymphome non Hodgkinien est un lymphome de Burkitt.

**10.** Combinaison pour une utilisation selon la revendication 3, dans laquelle le lymphome non Hodgkinien est un lymphome à cellules du manteau.

**11.** Combinaison selon la revendication 1 ou 2 pour une utilisation dans le traitement d'une leucémie lymphocytaire chronique.

**12.** Combinaison selon la revendication 1 ou la revendication 2, pour une utilisation dans le traitement d'une leucémie lymphoblastique aiguë.

**13.** Combinaison pour une utilisation selon l'une quelconque des revendications précédentes, dans laquelle l'anticorps spécifique du CD19 et la fludarabine sont :

(a) formulés ensemble dans une composition pharmaceutique ; ou
(b) formulés dans des compositions pharmaceutiques différentes.

**14.** Combinaison pour une utilisation selon l'une quelconque des revendications 1 à 13, dans laquelle ledit anticorps spécifique du CD19 et la fludarabine sont administrés simultanément.

**15.** Combinaison pour une utilisation selon l'une quelconque des revendications 1 à 12 ou 13(b), dans laquelle ledit anticorps spécifique du CD19 et la fludarabine sont administrés séparément.

**16.** Combinaison pour une utilisation selon la revendication 15, dans laquelle la fludarabine est administrée avant l'administration dudit anticorps spécifique du CD19.

# Figure 1

**Cytotoxicity of MOR208 and FLU alone and in combination**

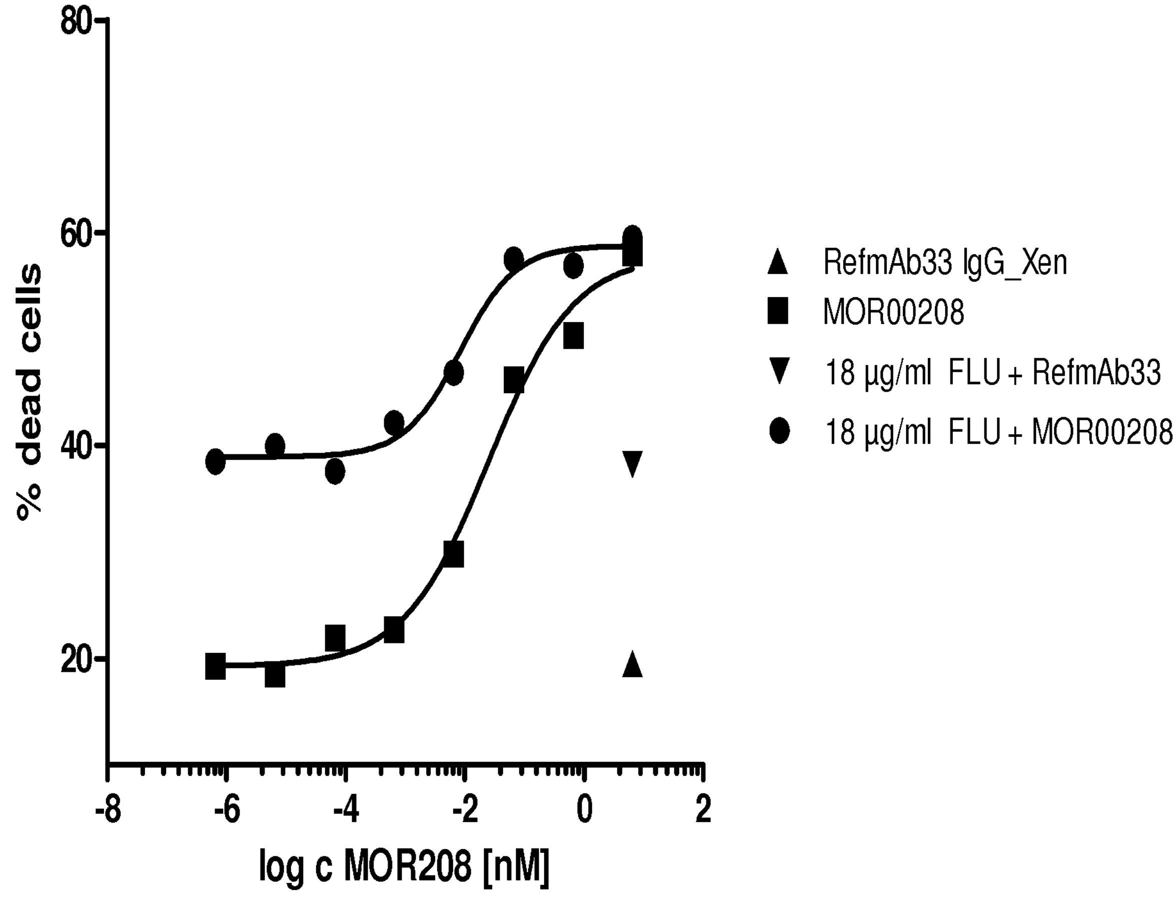

# Figure 2

## ADCC Dose response curves

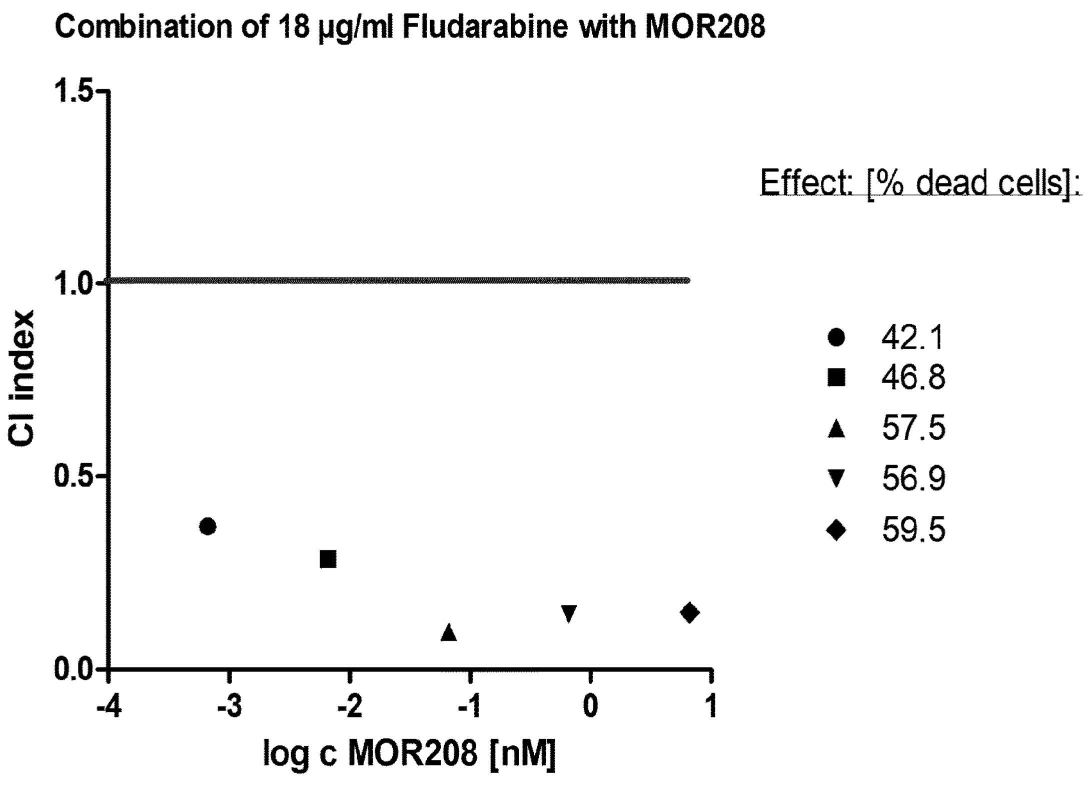

# Figure 3

The amino acid sequence of the MOR208 Variable Heavy Domain is:
(The CDRs are bolded and underlined)

EVQLVESGGGLVKPGGSLKLSCAASGYTFT**SYVMH**WVRQAPGKGLEWIGYI**NPY NDG**TKYNEKFQGRVTISSDKSISTAYMELSSLRSEDTAMYYCAR**GTYYYGTRVFDY**WG QGTLVTVSS (SEQ ID NO: 10)

The amino acid sequence of the MOR208 Variable Light Domain is:
(The CDRs are bolded and underlined)

DIVMTQSPATLSLSPGERATLSC**RSSKSLQNVNGNTYLY**WFQQKPGQSPQLLIY**R MSNLNS**GVPDRFSGSGSGTEFTLTISSLEPEDFAVYYC**MQHLEYPIT**FGAGTKLEIK (SEQ ID NO: 11)

The amino acid sequence of the MOR208 HCDR1 is: SYVMH (SEQ ID NO: 1)

The amino acid sequence of the MOR208 HCDR2 is: NPYNDG (SEQ ID NO: 2)

The amino acid sequence of the MOR208 HCDR3 is: GTYYYGTRVFDY (SEQ ID NO: 3)

The amino acid sequence of the MOR208 LCDR1 is: RSSKSLQNVNGNTYLY (SEQ ID NO: 4)

The amino acid sequence of the MOR208 LCDR2 is: RMSNLNS (SEQ ID NO: 5)

The amino acid sequence of the MOR208 LCDR3 is: MQHLEYPIT (SEQ ID NO: 6)

## Figure 4

## Sequence of Fc regions

The amino acids sequence of the MOR208 heavy chain Fc region is:

ASTKGPSVFPLAPSSKSTSGGTAALGCLVKD YFPEPVTVSWNSGALTSGVH
TFPAVLQSSGLYSLSSVVTVPSSSLGTQTYICNVNHKPSNTKVDKKVEPKSCDKTHTCPP
CPAPELLGGPDVFLFPPKPKDTLMISRTPEVTCVVVDVSHEDPEVQFNWYVDGVEVHNA
KTKPREEQFNSTFRVVSVLTVVHQDWLNGKEYKCKVSNKALPAPEEKTISKTKGQPREPQ
VYTLPPSREEMTKNQVSLTCLVKGFYPSDIAVEWESNGQPENNYKTTPPMLDSDGSFFLY
SKLTVDKSRWQQGNVFSCSVMHEALHNHYTQKSLSLSPGK (SEQ ID NO: 12)

The amino acids sequence of the MOR208 light chain Fc region is:

RTVAAPSVFIFPPSDEQLKSGTASVVCLLNNFYPREAKVQWKVDNALQSGNSQESVTEQ
DSKD STYSLSSTLTLSKADYEKHKVYACEVTHQGLSSPVTKSFNRGEC (SEQ ID NO: 13)

# Figure 5

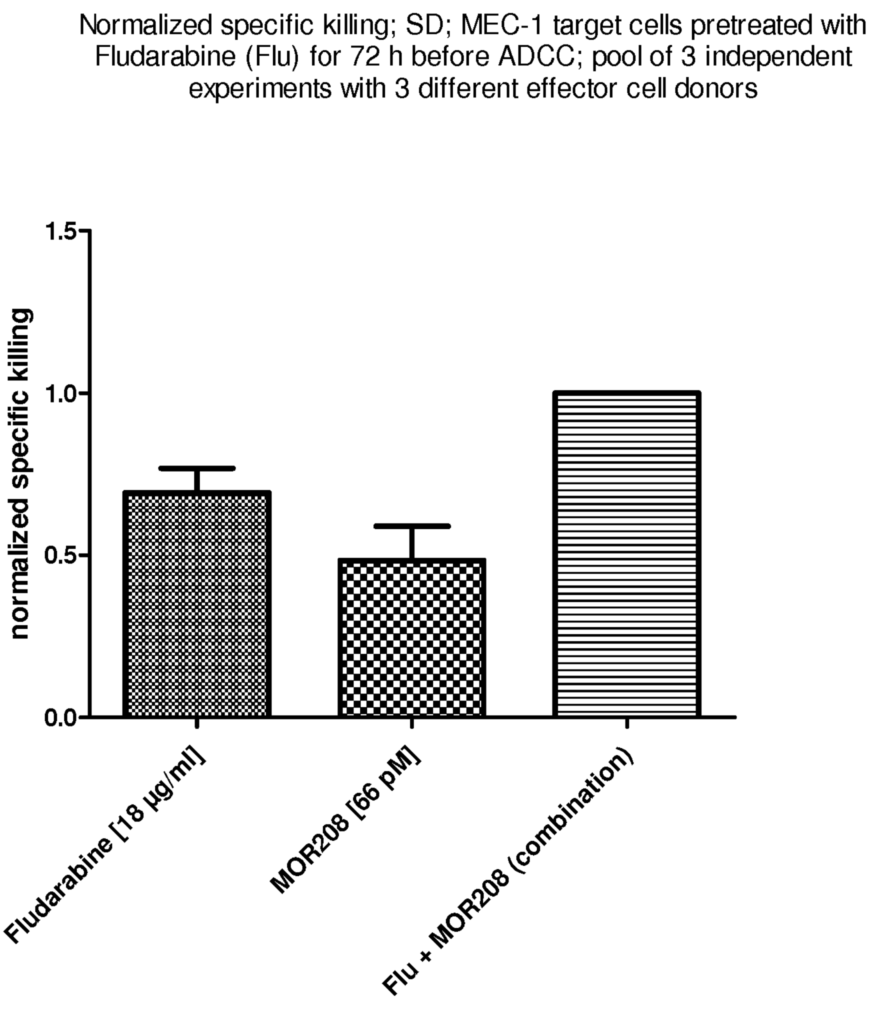


The figure shows the averages from the data shown in Table 2.

# Figure 6

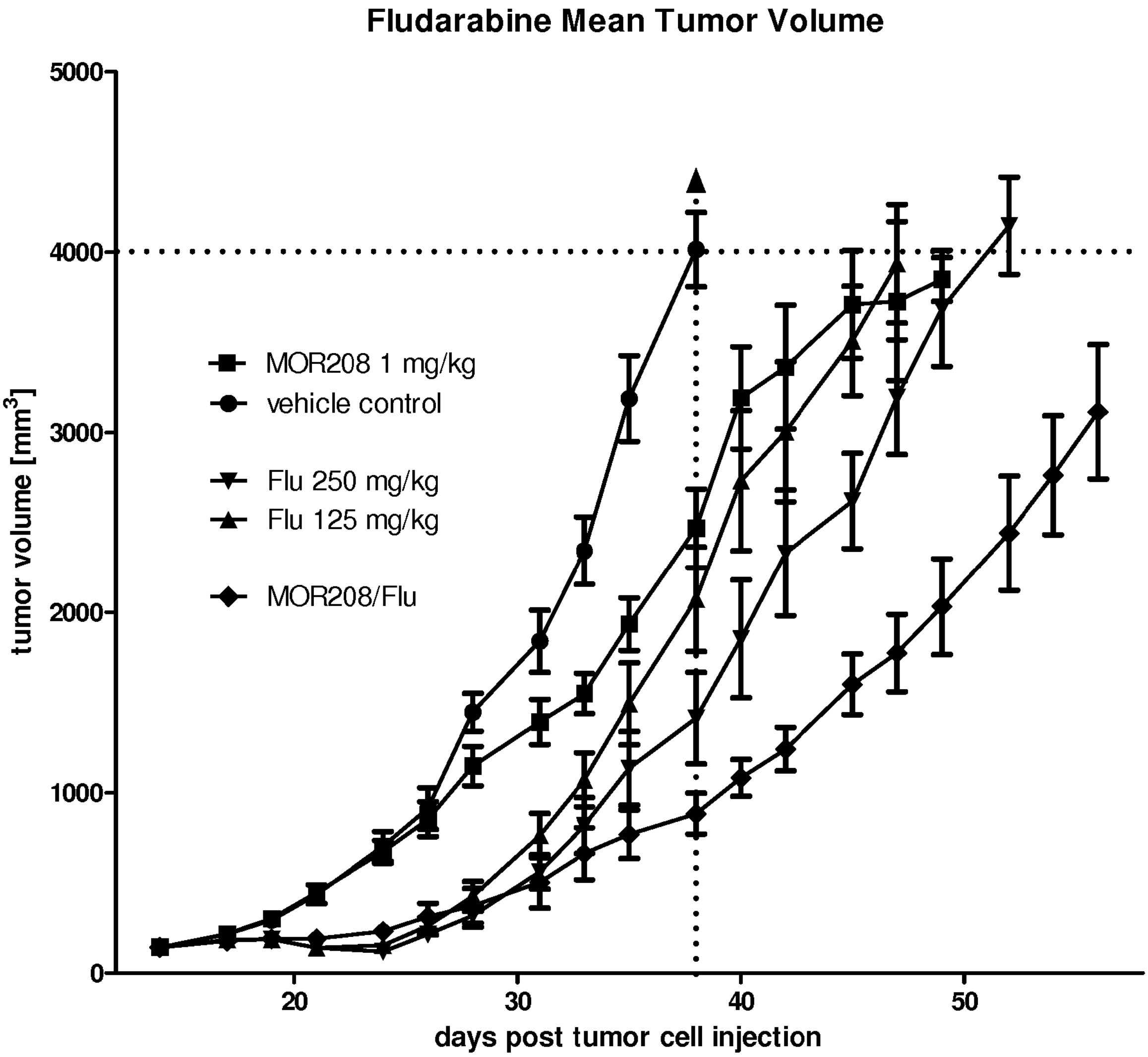
Fludarabine Mean Tumor Volume
5000
4000
3000
2000
1000
0
tumor volume [mm³]
MOR208 1 mg/kg
vehicle control
Flu 250 mg/kg
Flu 125 mg/kg
MOR208/Flu
20
30
40
50
days post tumor cell injection

Figure 7

Median Survival Time in SCID mouse model

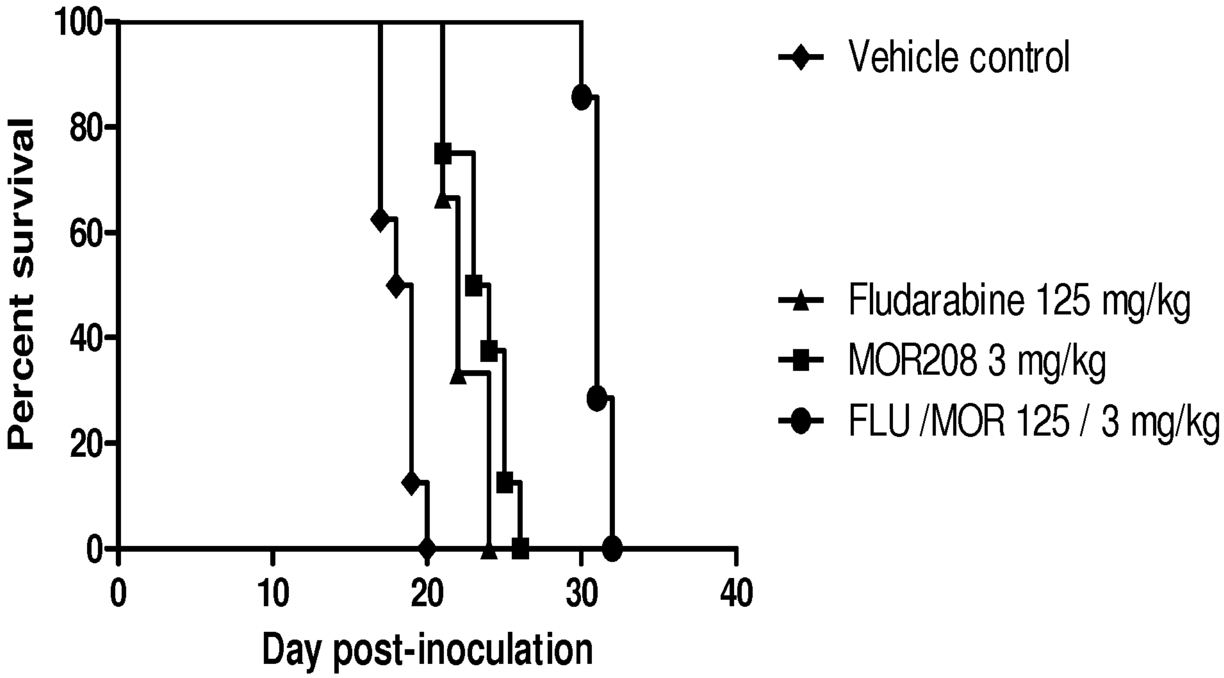

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011018225 A
- WO 2000074718 A
- US 201104150 B
- WO 2007076950 A
- WO 2005012493 A
- US 7109304 B
- WO 2010053716 A
- US 12266999 B
- WO 2007002223 A
- US 8097703 B
- WO 2008022152 A
- WO 12377251 A
- WO 2008150494 A
- WO 2008031056 A
- US 11852106 B
- US 648505 B
- WO 2009052431 A
- US 12253895 B
- WO 2010095031 A
- WO 12710442 A
- WO 2010151341 A
- US 13377514 B
- US 5686072 A
- WO 2002022212 A
- US 0129026 W
- US 377251
- US 917750
- US 852106
- US 648505
- US 7968687 B
- US 710442
- WO 200348731 A
- US 4708871 A
- EP 11177660
- US 61523862 B


### Non-patent literature cited in the description


- **NADLER et al.** *J. Immunol.,* 1983, vol. 131, 244-250
- **LOKEN et al.** *Blood,* 1987, vol. 70, 1316-1324
- **UCKUN et al.** *Blood,* 1988, vol. 71, 13-29
- **ANDERSON et al.** *Blood,* 1984, vol. 63, 1424-1433
- **SCHEUERMANN, LEUK.** *Lymphoma,* 1995, vol. 18, 385-397
- **GROSSBARD et al.** *Br. J. Haematol,* 1998, vol. 102, 509-15
- **TREON et al.** *Semin. Oncol,* 2003, vol. 30, 248-52
- **KOCHENDERFER et al.** Eradication of B lineage cell and regression of lymphoma in a patient treated with autologous T cells genetically engineered to recognize CD19. *Blood,* November 2010, vol. 116 (20
- The promise and potential pitfalls of chimeric antigen receptors. **SADELAIN et al.** Current Opinion in Immunology. Elsevier, 02 April 2009, vol. 21
- **ROSENBERG et al.** Treatment of B cell Malignancies with T cells expressing an anti-CD19 chimeric receptor. *Assessment of the Impact of Lymphocyte Depletion Prior to T cell Transfer,* September 2008
- **ESHHAR et al.** Proceedings of the National Academy of Sciences of USA. National Academy of Science, 15 January 1993, vol. 90
- Chronic lymphocytic leukemia treatment. **MONTSERRAT et al.** Blood Review. Churchill Livingstone, 01 September 1993, vol. 7
- **SCHEUERMANN et al.** CD19 Antigen in Leukemia and Lymphoma Diagnosis and Immunotherapy. *Leukemia and Lymphoma,* 1995, vol. 18, 385-397
- **TING-CHAO CHOU.** Theoretical Basis, Experimental Design, and Computerized Simulation of Synergism and Antagonism in Drug Combination Studies. *Pharmacol Rev,* 2006, vol. 58, 621-681
- **CLARKE et al.** Issues in experimental design and endpoint analysis in the study of experimental cytotoxic agents in vivo in breast cancer and other models. *Breast Cancer Research and Treatment,* 1997, vol. 46, 255-278
- **WEBB, J. L.** Enzyme and Metabolic Inhibitors. Academic Press, 1963
- **CHOTHIA C ; LESK AM.** Canonical structures for the hypervariable regions of immunoglobulins. *J Mol Biol.,* 1987, vol. 196 (4), 901-17
- **KABAT E.A ; WU T.T. ; PERRY H.M. ; GOTTESMAN K.S. ; FOELLER C.** Sequences of Proteins of Immunological Interest. US Dept. of Health and Human Services, 1991
- Epitope Mapping Protocols in Methods in Molecular Biology. Humana Press, 1996, vol. 66

- **GEYSEN et al.** *Proc. Natl. Acad. Sci. USA,* 1984, vol. 8, 3998-4002
- **GEYSEN et al.** *Proc. Natl. Acad. Sci. USA,* 1985, vol. 82, 78-182
- **GEYSEN et al.** *Mol. Immunol.,* 1986, vol. 23, 709-715
- **HOPP et al.** *Proc. Natl. Acad. Sci,* 1981
- **KYTE et al.** *J.Mol. Biol.,* 1982, vol. 157, 105-132
- **CHOU TC ; TALALAY P.** Quantitative analysis of dose-effect relationships: the combined effects of multiple drugs or enzyme inhibitors. *Adv Enzyme Regul,* 1984, vol. 22, 27-55
- **JAMES et al.** Antibody-mediated B-cell depletion before adoptive immunotherapy with T cells expressing CD20-specific chimeric T-cell receptors facilitates eradication of leukemia in immunocompetent mice. *Blood,* 30 October 2009, vol. 114 (27), 5454-63
- *Breast Cancer Research and Treatment,* 1997, vol. 46, 255-278

SEQUENCE LISTING

<110> MorphoSys AG

<120> Combinations and uses thereof

<150> EP11177660.5
<151> August 16, 2011

<150> US 61/523,862
<151> August 16, 2011

<160> 13

<170> BiSSAP 1.0

<210> 1
<211> 5
<212> PRT
<213> synthetic construct

<220>
<221> SOURCE
<222> 1..5
<223> /mol_type="protein" /note="HCDR1" /organism="synthetic construct"

<400> 1

Ser Tyr Val Met His
1 5

<210> 2
<211> 6
<212> PRT
<213> synthetic construct

<220>
<221> SOURCE
<222> 1..6
<223> /mol_type="protein" /note="HCDR2 " /organism="synthetic construct"

<400> 2

Asn Pro Tyr Asn Asp Gly
1 5

<210> 3
<211> 12
<212> PRT
<213> synthetic construct

<220>
<221> SOURCE
<222> 1..12
<223> /mol_type="protein" /note="HCDR3 " /organism="synthetic construct"

<400> 3

Gly Thr Tyr Tyr Tyr Gly Thr Arg Val Phe Asp Tyr
1 5 10

<210> 4
<211> 16
<212> PRT
<213> synthetic construct

<220>
<221> SOURCE
<222> 1..16
<223> /mol_type="protein" /note="LCDR1 " /organism="synthetic construct"

<400> 4

Arg Ser Ser Lys Ser Leu Gln Asn Val Asn Gly Asn Thr Tyr Leu Tyr
1 5 10 15

<210> 5
<211> 7
<212> PRT
<213> synthetic construct

<220>
<221> SOURCE
<222> 1..7
<223> /mol_type="protein" /note="LCDR2 " /organism="synthetic construct"

<400> 5

Arg Met Ser Asn Leu Asn Ser
1 5

<210> 6
<211> 9
<212> PRT
<213> synthetic construct

<220>
<221> SOURCE
<222> 1..9
<223> /mol_type="protein" /note="LCDR3 " /organism="synthetic construct"

<400> 6

Met Gln His Leu Glu Tyr Pro Ile Thr
1 5

<210> 7
<211> 556
<212> PRT
<213> Homo sapiens

<220>
<221> SOURCE
<222> 1..556
<223> /mol_type="protein" /note="CD19 " /organism="Homo sapiens"

<400> 7

Met Pro Pro Pro Arg Leu Leu Phe Phe Leu Leu Phe Leu Thr Pro Met
1 5 10 15
Glu Val Arg Pro Glu Glu Pro Leu Val Val Lys Val Glu Glu Gly Asp
20 25 30
Asn Ala Val Leu Gln Cys Leu Lys Gly Thr Ser Asp Gly Pro Thr Gln
35 40 45
Gln Leu Thr Trp Ser Arg Glu Ser Pro Leu Lys Pro Phe Leu Lys Leu
50 55 60
Ser Leu Gly Leu Pro Gly Leu Gly Ile His Met Arg Pro Leu Ala Ile
65 70 75 80
Trp Leu Phe Ile Phe Asn Val Ser Gln Gln Met Gly Gly Phe Tyr Leu
85 90 95
Cys Gln Pro Gly Pro Pro Ser Glu Lys Ala Trp Gln Pro Gly Trp Thr
100 105 110
Val Asn Val Glu Gly Ser Gly Glu Leu Phe Arg Trp Asn Val Ser Asp
115 120 125
Leu Gly Gly Leu Gly Cys Gly Leu Lys Asn Arg Ser Ser Glu Gly Pro
130 135 140
Ser Ser Pro Ser Gly Lys Leu Met Ser Pro Lys Leu Tyr Val Trp Ala
145 150 155 160
Lys Asp Arg Pro Glu Ile Trp Glu Gly Glu Pro Pro Cys Leu Pro Pro
165 170 175
Arg Asp Ser Leu Asn Gln Ser Leu Ser Gln Asp Leu Thr Met Ala Pro
180 185 190
Gly Ser Thr Leu Trp Leu Ser Cys Gly Val Pro Pro Asp Ser Val Ser
195 200 205
Arg Gly Pro Leu Ser Trp Thr His Val His Pro Lys Gly Pro Lys Ser
210 215 220
Leu Leu Ser Leu Glu Leu Lys Asp Asp Arg Pro Ala Arg Asp Met Trp
225 230 235 240
Val Met Glu Thr Gly Leu Leu Leu Pro Arg Ala Thr Ala Gln Asp Ala
245 250 255
Gly Lys Tyr Tyr Cys His Arg Gly Asn Leu Thr Met Ser Phe His Leu
260 265 270
Glu Ile Thr Ala Arg Pro Val Leu Trp His Trp Leu Leu Arg Thr Gly
275 280 285
Gly Trp Lys Val Ser Ala Val Thr Leu Ala Tyr Leu Ile Phe Cys Leu
290 295 300
Cys Ser Leu Val Gly Ile Leu His Leu Gln Arg Ala Leu Val Leu Arg
305 310 315 320
Arg Lys Arg Lys Arg Met Thr Asp Pro Thr Arg Arg Phe Phe Lys Val
325 330 335
Thr Pro Pro Pro Gly Ser Gly Pro Gln Asn Gln Tyr Gly Asn Val Leu
340 345 350
Ser Leu Pro Thr Pro Thr Ser Gly Leu Gly Arg Ala Gln Arg Trp Ala
355 360 365
Ala Gly Leu Gly Gly Thr Ala Pro Ser Tyr Gly Asn Pro Ser Ser Asp
370 375 380
Val Gln Ala Asp Gly Ala Leu Gly Ser Arg Ser Pro Pro Gly Val Gly
385 390 395 400
Pro Glu Glu Glu Glu Gly Glu Gly Tyr Glu Glu Pro Asp Ser Glu Glu
405 410 415
Asp Ser Glu Phe Tyr Glu Asn Asp Ser Asn Leu Gly Gln Asp Gln Leu
420 425 430
Ser Gln Asp Gly Ser Gly Tyr Glu Asn Pro Glu Asp Glu Pro Leu Gly
435 440 445
Pro Glu Asp Glu Asp Ser Phe Ser Asn Ala Glu Ser Tyr Glu Asn Glu
450 455 460
Asp Glu Glu Leu Thr Gln Pro Val Ala Arg Thr Met Asp Phe Leu Ser
465 470 475 480
Pro His Gly Ser Ala Trp Asp Pro Ser Arg Glu Ala Thr Ser Leu Gly
485 490 495

```
Ser Gln Ser Tyr Glu Asp Met Arg Gly Ile Leu Tyr Ala Ala Pro Gln
            500                     505                 510
Leu Arg Ser Ile Arg Gly Gln Pro Gly Pro Asn His Glu Glu Asp Ala
        515                     520                 525
Asp Ser Tyr Glu Asn Met Asp Asn Pro Asp Gly Pro Asp Pro Ala Trp
    530                     535                 540
Gly Gly Gly Gly Arg Met Gly Thr Trp Ser Thr Arg
545                     550                 555
```

<210> 8
<211> 450
<212> PRT
<213> synthetic construct

<220>
<221> SOURCE
<222> 1..450
<223> /mol_type="protein" /note="Heavy chain RefmAb33" /organism="synthetic construct"

<400> 8

```
Gln Val Thr Leu Arg Glu Ser Gly Pro Ala Leu Val Lys Pro Thr Gln
1               5                   10                  15
Thr Leu Thr Leu Thr Cys Thr Phe Ser Gly Phe Ser Leu Ser Thr Ala
            20                  25                  30
Gly Met Ser Val Gly Trp Ile Arg Gln Pro Pro Gly Lys Ala Leu Glu
        35                  40                  45
Trp Leu Ala Asp Ile Trp Trp Asp Asp Lys Lys His Tyr Asn Pro Ser
    50                  55                  60
Leu Lys Asp Arg Leu Thr Ile Ser Lys Asp Thr Ser Lys Asn Gln Val
65                  70                  75                  80
Val Leu Lys Val Thr Asn Met Asp Pro Ala Asp Thr Ala Thr Tyr Tyr
                85                  90                  95
Cys Ala Arg Asp Met Ile Phe Asn Phe Tyr Phe Asp Val Trp Gly Gln
            100                 105                 110
Gly Thr Thr Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Lys Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Asp Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Gln Phe Asn Trp Tyr Val Asp Gly Val Glu Val His
        275                 280                 285
Asn Ala Lys Thr Lys Pro Arg Glu Glu Gln Phe Asn Ser Thr Phe Arg
    290                 295                 300
Val Val Ser Val Leu Thr Val Val His Gln Asp Trp Leu Asn Gly Lys
305                 310                 315                 320
Glu Tyr Lys Cys Lys Val Ser Asn Lys Ala Leu Pro Ala Pro Glu Glu


                325                 330                 335
Lys Thr Ile Ser Lys Thr Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Met
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Lys
    450
```

<210> 9

<211> 213
<212> PRT
<213> synthetic construct

<220>
<221> SOURCE
<222> 1..213
<223> /mol_type="protein" /note="Light chain RefmAb33" /organism="synthetic construct"

<400> 9

```
Asp Ile Gln Met Thr Gln Ser Pro Ser Thr Leu Ser Ala Ser Val Gly
1               5                   10                  15
Asp Arg Val Thr Ile Thr Cys Ser Ala Ser Ser Arg Val Gly Tyr Met
            20                  25                  30
His Trp Tyr Gln Gln Lys Pro Gly Lys Ala Pro Lys Leu Leu Ile Tyr
        35                  40                  45
Asp Thr Ser Lys Leu Ala Ser Gly Val Pro Ser Arg Phe Ser Gly Ser
    50                  55                  60
Gly Ser Gly Thr Glu Phe Thr Leu Thr Ile Ser Ser Leu Gln Pro Asp
65                  70                  75                  80
Asp Phe Ala Thr Tyr Tyr Cys Phe Gln Gly Ser Gly Tyr Pro Phe Thr
                85                  90                  95
Phe Gly Gly Gly Thr Lys Val Glu Ile Lys Arg Thr Val Ala Ala Pro
            100                 105                 110
Ser Val Phe Ile Phe Pro Pro Ser Asp Glu Gln Leu Lys Ser Gly Thr
        115                 120                 125
Ala Ser Val Val Cys Leu Leu Asn Asn Phe Tyr Pro Arg Glu Ala Lys
    130                 135                 140
Val Gln Trp Lys Val Asp Asn Ala Leu Gln Ser Gly Asn Ser Gln Glu
145                 150                 155                 160
Ser Val Thr Glu Gln Asp Ser Lys Asp Ser Thr Tyr Ser Leu Ser Ser
                165                 170                 175
Thr Leu Thr Leu Ser Lys Ala Asp Tyr Glu Lys His Lys Val Tyr Ala
            180                 185                 190
Cys Glu Val Thr His Gln Gly Leu Ser Ser Pro Val Thr Lys Ser Phe
        195                 200                 205
Asn Arg Gly Glu Cys
    210
```

<210> 10
<211> 121
<212> PRT
<213> synthetic construct

<220>
<221> SOURCE
<222> 1..121
<223> /mol_type="protein" /note="heavy chain " /organism="synthetic construct"

<400> 10

```
Glu Val Gln Leu Val Glu Ser Gly Gly Gly Leu Val Lys Pro Gly Gly
1               5                   10                  15
Ser Leu Lys Leu Ser Cys Ala Ala Ser Gly Tyr Thr Phe Thr Ser Tyr
            20                  25                  30
Val Met His Trp Val Arg Gln Ala Pro Gly Lys Gly Leu Glu Trp Ile
        35                  40                  45
Gly Tyr Ile Asn Pro Tyr Asn Asp Gly Thr Lys Tyr Asn Glu Lys Phe
    50                  55                  60
Gln Gly Arg Val Thr Ile Ser Ser Asp Lys Ser Ile Ser Thr Ala Tyr
65                  70                  75                  80
Met Glu Leu Ser Ser Leu Arg Ser Glu Asp Thr Ala Met Tyr Tyr Cys
                85                  90                  95
Ala Arg Gly Thr Tyr Tyr Tyr Gly Thr Arg Val Phe Asp Tyr Trp Gly
            100                 105                 110
Gln Gly Thr Leu Val Thr Val Ser Ser
        115                 120
```

<210> 11
<211> 112
<212> PRT
<213> synthetic construct

<220>
<221> SOURCE
<222> 1..112
<223> /mol_type="protein" /note=" light chain " /organism="synthetic construct"

<400> 11

```
Asp Ile Val Met Thr Gln Ser Pro Ala Thr Leu Ser Leu Ser Pro Gly
1               5                   10                  15
Glu Arg Ala Thr Leu Ser Cys Arg Ser Ser Lys Ser Leu Gln Asn Val
            20                  25                  30
Asn Gly Asn Thr Tyr Leu Tyr Trp Phe Gln Gln Lys Pro Gly Gln Ser
        35                  40                  45
Pro Gln Leu Leu Ile Tyr Arg Met Ser Asn Leu Asn Ser Gly Val Pro
    50                  55                  60
Asp Arg Phe Ser Gly Ser Gly Ser Gly Thr Glu Phe Thr Leu Thr Ile
65                  70                  75                  80
Ser Ser Leu Glu Pro Glu Asp Phe Ala Val Tyr Tyr Cys Met Gln His
                85                  90                  95
Leu Glu Tyr Pro Ile Thr Phe Gly Ala Gly Thr Lys Leu Glu Ile Lys
            100                 105                 110
```

<210> 12
<211> 329
<212> PRT
<213> synthetic construct

<220>
<221> SOURCE
<222> 1..329
<223> /mol_type="protein" /note="heavy chain constant domain" /organism="synthetic construct"

<400> 12

Ala Ser Thr Lys Gly Pro Ser Val Phe Pro Leu Ala Pro Ser Ser Lys
1 5 10 15
Ser Thr Ser Gly Gly Thr Ala Ala Leu Gly Cys Leu Val Lys Asp Tyr
20 25 30
Phe Pro Glu Pro Val Thr Val Ser Trp Asn Ser Gly Ala Leu Thr Ser
35 40 45
Gly Val His Thr Phe Pro Ala Val Leu Gln Ser Ser Gly Leu Tyr Ser
50 55 60
Leu Ser Ser Val Val Thr Val Pro Ser Ser Ser Leu Gly Thr Gln Thr
65 70 75 80
Tyr Ile Cys Asn Val Asn His Lys Pro Ser Asn Thr Lys Val Asp Lys
85 90 95
Lys Val Glu Pro Lys Ser Cys Asp Lys Thr His Thr Cys Pro Pro Cys
100 105 110
Pro Ala Pro Glu Leu Leu Gly Gly Pro Asp Val Phe Leu Phe Pro Pro
115 120 125
Lys Pro Lys Asp Thr Leu Met Ile Ser Arg Thr Pro Glu Val Thr Cys
130 135 140
Val Val Val Asp Val Ser His Glu Asp Pro Glu Val Gln Phe Asn Trp
145 150 155 160
Tyr Val Asp Gly Val Glu Val His Asn Ala Lys Thr Lys Pro Arg Glu
165 170 175
Glu Gln Phe Asn Ser Thr Phe Arg Val Val Ser Val Leu Thr Val Val
180 185 190
His Gln Asp Trp Leu Asn Gly Lys Glu Tyr Lys Cys Lys Val Ser Asn
195 200 205
Lys Ala Leu Pro Ala Pro Glu Glu Lys Thr Ile Ser Lys Thr Lys Gly
210 215 220
Gln Pro Arg Glu Pro Gln Val Tyr Thr Leu Pro Pro Ser Arg Glu Glu
225 230 235 240
Met Thr Lys Asn Gln Val Ser Leu Thr Cys Leu Val Lys Gly Phe Tyr
245 250 255
Pro Ser Asp Ile Ala Val Glu Trp Glu Ser Asn Gly Gln Pro Glu Asn
260 265 270
Asn Tyr Lys Thr Thr Pro Pro Met Leu Asp Ser Asp Gly Ser Phe Phe
275 280 285
Leu Tyr Ser Lys Leu Thr Val Asp Lys Ser Arg Trp Gln Gln Gly Asn
290 295 300
Val Phe Ser Cys Ser Val Met His Glu Ala Leu His Asn His Tyr Thr
305 310 315 320
Gln Lys Ser Leu Ser Leu Ser Pro Gly Lys
325 330

<210> 13
<211> 107
<212> PRT
<213> synthetic construct

<220>
<221> SOURCE
<222> 1..107
<223> /mol_type="protein" /note="light chain constant domain" /organism="synthetic construct"

<400> 13

Arg Thr Val Ala Ala Pro Ser Val Phe Ile Phe Pro Pro Ser Asp Glu
1 5 10 15

```
Gln Leu Lys Ser Gly Thr Ala Ser Val Val Cys Leu Leu Asn Asn Phe
            20                  25                  30
Tyr Pro Arg Glu Ala Lys Val Gln Trp Lys Val Asp Asn Ala Leu Gln
        35                  40                  45
Ser Gly Asn Ser Gln Glu Ser Val Thr Glu Gln Asp Ser Lys Asp Ser
    50                  55                  60
Thr Tyr Ser Leu Ser Ser Thr Leu Thr Leu Ser Lys Ala Asp Tyr Glu
65                  70                  75                  80
Lys His Lys Val Tyr Ala Cys Glu Val Thr His Gln Gly Leu Ser Ser
                85                  90                  95
Pro Val Thr Lys Ser Phe Asn Arg Gly Glu Cys
            100                 105
```